# EUROPEAN PATENT APPLICATION

(11) **EP 1 316 560 A1**
(43) Date of publication of application: **04.06.2003**
(21) Application number: 01963516.8
(22) Date of filing: 07.09.2001
(51) Int. Cl.: C07K 7/06, C07K 14/82, C07K 16/32, C07K 19/00, A61K 38/00, A61K 39/395, A61K 45/00, A61P 35/00, A61P 37/04, A61P 37/06, G01N 33/15, G01N 33/50

(54) **NOVEL HUMAN CANCER/TESTIS ANTIGEN AND GENE THEREOF**

(30) Priority: 08.09.2000 JP 2000274218
(71) Applicant: JAPAN SCIENCE AND TECHNOLOGY CORPORATION, Kawaguchi-shi, Saitama 332-0012 (JP)
(72) Inventor: TAKIMOTO, Masato, Sapporo-shi, Hokkaido 062-0008 (JP); KUZUMAKI, Noboru, Sapporo-shi, Hokkaido 063-0850 (JP); SATO, Noriyuki, Sapporo-shi, Hokkaido 062-0042 (JP); SAHARA, Hiroeki, Sapporo-shi, Hokkaido 005-0855 (JP)
(74) Representative: Denholm, Anna Marie
(86) International application number: JP0107784
(87) International publication number: WO02020560

(57) **Abstract**

The present invention provides a cancer/testis antigen which is applicable to diagnosis and immunotherapy for cancer, that is to say, to provide a cancer/testis antigen which is not expressed in normal tissues other than the testis, which is expressed in various cancers over a broad range and which induces immune response in a host, and to provide a caner/testis gene encoding the cancer/testis antigen, etc. By using the yeast Two-hybrid System, the present inventors screened a protein which specifically binds to GCF, a previously reported transcription factor, cloned a gene of a protein specifically binding to the GCF, confirmed that the D40 protein as a resulting gene product is a cancer/testis antigen which is expressed substantially only in the testis in normal human tissues whereas in cancers it is expressed in human primary cancers over a broad range derived from various tissues and cells, and found that the D40 shows high affinity with HLA and has a plurality of sequences comprising 9 or 10 amino acid residues and binding to HLA.

SEQUENCE LISTING

<110> JAPAN SCIENCE AND TECHNOLOGY CORPORATION

<120> A novel human cancer/testis-associated gene

<130> YG13-22PCT

<140>
<141>

<150> JP 2000/274218
<151> 2000-09-08

<160> 227

<170> PatentIn Ver. 2.1

<210> 1
<211> 5412
<212> DNA
<213> Homo sapiens

<400> 1
gagatttgaa tacttcactg aggcgagccg ggcgttgtga gcggactgct agaggcggct 60
gtctgtttcc gctctaagga aactcagagc gtgtggaccc caaacaagtc tgcgcaaaat 120
ttgtcgagga ggtttgccgc ggcagaaaag ttttcttcaa aaatggatgg ggtgtcttca 180
gaggctaatg aagaaaatga caatatagag agacctgtta gaagacggca ttcttcaata 240
ttgaaacccc caaggagtcc tcttcaggac ctcagaggtg ggaatgaaac agttcaggaa 300
tccaatgctt tgagaaataa gaaaaactct cgtcgagtca gctttgcaga tactataaag 360

gtattccaga cggagtctca tatgaaaata gtgagaaagt cagaaatgga agaaacagaa 420
gcaggagaaa atcttctttt gatacagaat aagaaattag aagataatta ctgtgaaatt 480
actgggatga acacattgct ttctgctccc attcataccc agatgcaaca gaaggagttt 540
tcaattatag aacatacccg tgaaaggaaa catgcaaatg accagacagt cattttttca 600
gatgaaaacc agatggacct gacatcaagt cacactgtaa tgattaccaa aggcctttta 660
gataatccca taagtgaaaa gtccaccaag atagatacca catcatttct agctaattta 720
aagcttcaca ccgaggactc aagaatgaaa aaagaagtaa atttttccgt ggatcaaaac 780
acttcttcag aaaataaaat agatttcaat gacttcataa aaagattgaa aacaggaaaa 840
tgtagtgctt ttcctgatgt gcctgataaa gaaaattttg agatacctat ttattccaag 900
gaaccgaaca gtgcctcttc tacacatcaa atgcatgtat ctcttaagga agatgaaaat 960
aacagtaata ttactaggct ctttagagaa aaagatgatg ggatgaattt cacccagtgt 1020
catacagcca atattcagac attgattccc acatccagtg agaccaactc acgggaatct 1080
aaaggtaatg atattacaat ttatggcaat gactttatgg acttgacatt taaccacact 1140
ttgcagatct tacctgcaac aggtaatttt tctgaaatag aaaatcaaac tcagaatgcc 1200
atggatgtaa caacaggtta tggaactaaa gcttcaggaa ataaaacagt ttttaagagt 1260
aaacaaaata ctgcttttca agacctttcc ataaactctg cagacaaaat acatattacc 1320
agaagtcata ttatgggggc agaaactcac atagtctcac agacttgtaa tcaggatgcc 1380
agaatattag ccatgacccc agaatctata tattctaatc catctattca aggttgtaag 1440
actgttttct attctagttg taatgatgcc atggaaatga ccaaatgtct ctcaaatatg 1500
agagaggaga aaaatttgct aaagcatgac agtaattatt ctaaaatgta ttgcaatcca 1560
gatgctatgt cttctctcac agagaaaact atttattccg gagaggagaa catggacatt 1620
accaagagtc atacagttgc aatagataat caaattttta aacaagatca atcaaatgtg 1680
caaatagcag ctgcaccaac acccgaaaaa gaaatgatgc tccaaaatct tatgaccaca 1740
tcagaagatg ggaaaatgaa tgtaaattgt aactcagttc ctcatgtatc taaggaaaga 1800
atacagcaga gcctgtcaaa tcctttgtct atttcattga ctgatagaaa gactgaactc 1860
ttatcaggtg aaaatacgga tttgactgaa agtcacacaa gtaacttagg aagtcaggtt 1920
cctcttgcag cttataatct agcaccggag agtaccagtg aatctcactc tcagagcaaa 1980
agctcttcag atgaatgtga agaaattacc aaaagtcgta atgaaccatt tcagcgatca 2040
gacataatag ccaaaaacag cttaaccgac acctggaaca aagacaaaga ttgggttttg 2100
aagattttgc cctaccttga taaagattct cctcagtcag ctgattgtaa tcaggagata 2160

gcaacaagcc ataatatagt ctactgtggt ggagttcttg ataaacaaat aactaataga 2220
aatacagtat catgggaaca atctttgttt tctaccacaa agccattatt ttcatcagga 2280
cagttctcta tgaaaaatca tgatactgct ataagtagtc atacagtgaa atctgtacta 2340
ggccagaatt ctaaactggc tgagccactg aggaaaagtt taagcaatcc cacacctgac 2400
tattgccatg acaagatgat tatatgttca gaggaagagc aaaatatgga tctaacaaag 2460
agccacactg ttgtcattgg atttggtcct tctgaactac aagaacttgg taaaactaat 2520
ttagaacaca ctactggcca gctaacaaca atgaacagac agatagctgt aaaagttgaa 2580
aaatgtggta aaagtcccat agaaaaaagt ggagtgctta aatctaactg tattatggat 2640
gtgttagagg acgaaagtgt acagaaacct aaatttccaa aggaaaagca aaatgtcaaa 2700
atttggggaa ggaaaagtgt tggtggacca aaaattgata agacgattgt attttcagaa 2760
gacgataaga atgatatgga tatcactaag agttatacaa tagaaataaa ccatagactt 2820
tattagagaa acgtgattgt catttggtgc cattggcagg aacttctgaa actattttat 2880
atacatgtgg gcaggatgac atggagatca ctagaagtca cacaactgcc ttagaatgta 2940
aaactgtctc accagatgaa ataactacta ggcctatgga caaaactgta gtgtttgtag 3000
ataatcatgt tgaactagaa atgacagagt cccatactgt tttcattgac taccaagaaa 3060
aggaaagaac agacagacct aactttgaac tatcccaaag gaaaagccta ggaacaccaa 3120
cagtgatatg tactcctact gaggagagtg ttttctttcc aggaaatggt gaaagtgacc 3180
gtctagtagc aaatgacagc cagctaaccc ctctggagga atggtctaat aataggggcc 3240
ctgtagaggt agctgataac atggaattgt ctaaatcagc cacttgcaaa aacatcaaag 3300
atgtacaaag tcctggattt ctgaatgaac ctctatcaag caaaagtcag agaagaaaaa 3360
gccttaagct aaaaaatgac aagaccattg tattttcaga gaatcataaa aatgatatgg 3420
atattaccca gagttgtatg gtggaaatag ataacgaaag tgccctggag gataaagagg 3480
acttccattt ggcaggggct tctaaaacta ttttgtattc atgtgggcag gatgacatgg 3540
agatcactag gagtcacaca actgccttag aatgtaaaac tctcctgcca aacgaaatag 3600
ctattaggcc catggacaaa accgtattgt tcacagataa ttacagtgat ctggaagtca 3660
ccgattccca tactgttttc attgactgtc aagccacaga gaaaatactt gaagaaaacc 3720
ctaaatttgg aataggaaaa ggaaaaaact tgggtgtttc ctttcctaag gataatagct 3780
gtgttcaaga aatcgctgaa aaacaagcac tggctgtagg aaacaaaata gttcttcaca 3840
ccgagcaaaa gcaacaactc tttgctgcta ctaatagaac tactaatgaa atcatcaaat 3900
ttcatagtgc tgctatggat gaaaaggtca tagggaaagt tgtagaccag gcctgtacat 3960

tggaaaaagc gcaagttgaa agctgtcagt taaataatag agatagaaga aatgtggact 4020
ttacaagtag tcatgcaact gctgtttgtg gatccagtga taattattcc tgtttagcaa 4080
atgttatttc ctgtactgat aatttggagg gtagtgccat gctcttatgt gataaagatg 4140
aggaaaaagc ccattattgc ccagtgcaaa atgatcttgc ttatgcaaat gattttgcca 4200
gtgaatatta cttggaatct gagggacagc ctctctctgc tccttgtcct ttgttagaga 4260
aggaagaagt tattcaaacc agtaccaaag gacagttaga ctgtgttata acactgcaca 4320
aagatcaaga tctgattaag gatccacgaa atctattggc taatcaaact ttagtatata 4380
gtcaagatct gggggagatg actaaactta attcaaagcg agtatctttt aagcttccaa 4440
aggatcaaat gaaagtctat gttgatgaca tttatgttat tcctcagcct catttctcaa 4500
ccgaccaacc tccattacct aaaaaaggac agagtagtat caataaagaa gaagtaatac 4560
tgtctaaagc tggaaataag agtttaaata ttatagaaaa ttcctctgca cccatatgtg 4620
aaaacaagcc caaaatactc aatagtgagg aatggtttgc tgcagcctgt aaaaaagaac 4680
tgaaggaaaa tattcaaaca actaactata atacagctct agatttccac agtaactcag 4740
acgtaactaa gcaagtcatt caaactcatg tcaatgctgg agaagcacca gatcctgtaa 4800
ttacatctaa tgttccatgt tttcatagta tcaaaccaaa tctgaataat ttgaatggaa 4860
aaactggaga gtttttagcc tttcaaactg ttcatctacc accccttcca gagcaattac 4920
ttgaattagg aaataaggca cacaatgata tgcatatagt gcaagctaca gaaatacata 4980
atattaacat aatctccagc aatgctaaag atagtagaga tgaggaaaat aaaaagtctc 5040
ataatggagc tgaaaccacc tctctaccgc caaagacagt ttttaaagat aaagtaagga 5100
gatgttcttt gggaatcttt ttgcctagat tgcccaacaa gagaaattgt agtgtcactg 5160
gtattgatga cctggaacag attccagcag acacaactga tataaatcac ttagaaactc 5220
agccggtctc tagcaaagat tcaggcattg gatctgttgc aggtaaactg aacctaagtc 5280
cttctcaata tataaatgag gaaaatcttc ctgtatatcc tgatgagatc aattcttcag 5340
actctattta catagaaact gaggaaaagg ccgtgggtac aagaagaaga agatattcat 5400
aaggagaaaa aa 5412

<210> 2
<211> 887
<212> PRT

<213> Homo sapiens

<400> 2

Met Asp Gly Val Ser Ser Glu Ala Asn Glu Glu Asn Asp Asn Ile Glu
1               5                   10                  15

Arg Pro Val Arg Arg Arg His Ser Ser Ile Leu Lys Pro Pro Arg Ser
            20                  25                  30

Pro Leu Gln Asp Leu Arg Gly Gly Asn Glu Thr Val Gln Glu Ser Asn
        35                  40                  45

Ala Leu Arg Asn Lys Lys Asn Ser Arg Arg Val Ser Phe Ala Asp Thr
    50                  55                  60

Ile Lys Val Phe Gln Thr Glu Ser His Met Lys Ile Val Arg Lys Ser
65                  70                  75                  80

Glu Met Glu Glu Thr Glu Ala Gly Glu Asn Leu Leu Leu Ile Gln Asn
                85                  90                  95

Lys Lys Leu Glu Asp Asn Tyr Cys Glu Ile Thr Gly Met Asn Thr Leu
            100                 105                 110

Leu Ser Ala Pro Ile His Thr Gln Met Gln Gln Lys Glu Phe Ser Ile
        115                 120                 125

Ile Glu His Thr Arg Glu Arg Lys His Ala Asn Asp Gln Thr Val Ile
    130                 135                 140

Phe Ser Asp Glu Asn Gln Met Asp Leu Thr Ser Ser His Thr Val Met
145                 150                 155                 160

Ile Thr Lys Gly Leu Leu Asp Asn Pro Ile Ser Glu Lys Ser Thr Lys
                165                 170                 175

Ile Asp Thr Thr Ser Phe Leu Ala Asn Leu Lys Leu His Thr Glu Asp
            180                 185                 190

Ser Arg Met Lys Lys Glu Val Asn Phe Ser Val Asp Gln Asn Thr Ser
        195                 200                 205

Ser Glu Asn Lys Ile Asp Phe Asn Asp Phe Ile Lys Arg Leu Lys Thr
    210                 215                 220

Gly Lys Cys Ser Ala Phe Pro Asp Val Pro Asp Lys Glu Asn Phe Glu
225                 230                 235                 240

Ile Pro Ile Tyr Ser Lys Glu Pro Asn Ser Ala Ser Ser Thr His Gln
                245                 250                 255

Met His Val Ser Leu Lys Glu Asp Glu Asn Asn Ser Asn Ile Thr Arg
            260                 265                 270

Leu Phe Arg Glu Lys Asp Asp Gly Met Asn Phe Thr Gln Cys His Thr
        275                 280                 285

Ala Asn Ile Gln Thr Leu Ile Pro Thr Ser Ser Glu Thr Asn Ser Arg
    290                 295                 300

Glu Ser Lys Gly Asn Asp Ile Thr Ile Tyr Gly Asn Asp Phe Met Asp
305                 310                 315                 320

Leu Thr Phe Asn His Thr Leu Gln Ile Leu Pro Ala Thr Gly Asn Phe
                325                 330                 335

Ser Glu Ile Glu Asn Gln Thr Gln Asn Ala Met Asp Val Thr Thr Gly
            340                 345                 350

Tyr Gly Thr Lys Ala Ser Gly Asn Lys Thr Val Phe Lys Ser Lys Gln
        355                 360                 365

Asn Thr Ala Phe Gln Asp Leu Ser Ile Asn Ser Ala Asp Lys Ile His
    370                 375                 380

Ile Thr Arg Ser His Ile Met Gly Ala Glu Thr His Ile Val Ser Gln
385                 390                 395                 400

Thr Cys Asn Gln Asp Ala Arg Ile Leu Ala Met Thr Pro Glu Ser Ile
                405                 410                 415

Tyr Ser Asn Pro Ser Ile Gln Gly Cys Lys Thr Val Phe Tyr Ser Ser
            420                 425                 430

Cys Asn Asp Ala Met Glu Met Thr Lys Cys Leu Ser Asn Met Arg Glu
        435                 440                 445

Glu Lys Asn Leu Leu Lys His Asp Ser Asn Tyr Ser Lys Met Tyr Cys
    450                 455                 460

Asn Pro Asp Ala Met Ser Ser Leu Thr Glu Lys Thr Ile Tyr Ser Gly
465                 470                 475                 480

Glu Glu Asn Met Asp Ile Thr Lys Ser His Thr Val Ala Ile Asp Asn
                485                 490                 495

Gln Ile Phe Lys Gln Asp Gln Ser Asn Val Gln Ile Ala Ala Ala Pro
            500                 505                 510

Thr Pro Glu Lys Glu Met Met Leu Gln Asn Leu Met Thr Thr Ser Glu
        515                 520                 525

Asp Gly Lys Met Asn Val Asn Cys Asn Ser Val Pro His Val Ser Lys
    530                 535                 540

Glu Arg Ile Gln Gln Ser Leu Ser Asn Pro Leu Ser Ile Ser Leu Thr
545                 550                 555                 560

Asp Arg Lys Thr Glu Leu Leu Ser Gly Glu Asn Thr Asp Leu Thr Glu
                565                 570                 575

Ser His Thr Ser Asn Leu Gly Ser Gln Val Pro Leu Ala Ala Tyr Asn
            580                 585                 590

Leu Ala Pro Glu Ser Thr Ser Glu Ser His Ser Gln Ser Lys Ser Ser
        595                 600                 605

Ser Asp Glu Cys Glu Glu Ile Thr Lys Ser Arg Asn Glu Pro Phe Gln
    610                 615                 620

Arg Ser Asp Ile Ile Ala Lys Asn Ser Leu Thr Asp Thr Trp Asn Lys
625                 630                 635                 640

Asp Lys Asp Trp Val Leu Lys Ile Leu Pro Tyr Leu Asp Lys Asp Ser
                645                 650                 655

Pro Gln Ser Ala Asp Cys Asn Gln Glu Ile Ala Thr Ser His Asn Ile
            660                 665                 670

Val Tyr Cys Gly Gly Val Leu Asp Lys Gln Ile Thr Asn Arg Asn Thr
        675                 680                 685

Val Ser Trp Glu Gln Ser Leu Phe Ser Thr Thr Lys Pro Leu Phe Ser
    690                 695                 700

Ser Gly Gln Phe Ser Met Lys Asn His Asp Thr Ala Ile Ser Ser His
705                 710                 715                 720

Thr Val Lys Ser Val Leu Gly Gln Asn Ser Lys Leu Ala Glu Pro Leu
                725                 730                 735

Arg Lys Ser Leu Ser Asn Pro Thr Pro Asp Tyr Cys His Asp Lys Met
            740                 745                 750

Ile Ile Cys Ser Glu Glu Glu Gln Asn Met Asp Leu Thr Lys Ser His
        755                 760                 765

Thr Val Val Ile Gly Phe Gly Pro Ser Glu Leu Gln Glu Leu Gly Lys
    770                 775                 780

Thr Asn Leu Glu His Thr Thr Gly Gln Leu Thr Thr Met Asn Arg Gln
785                 790                 795                 800

Ile Ala Val Lys Val Glu Lys Cys Gly Lys Ser Pro Ile Glu Lys Ser
                805                 810                 815

Gly Val Leu Lys Ser Asn Cys Ile Met Asp Val Leu Glu Asp Glu Ser
            820                 825                 830

Val Gln Lys Pro Lys Phe Pro Lys Glu Lys Gln Asn Val Lys Ile Trp
        835                 840                 845

Gly Arg Lys Ser Val Gly Gly Pro Lys Ile Asp Lys Thr Ile Val Phe
    850                 855                 860

Ser Glu Asp Asp Lys Asn Asp Met Asp Ile Thr Lys Ser Tyr Thr Ile
865                 870                 875                 880

Glu Ile Asn His Arg Leu Tyr
                885

<210> 3
<211> 833
<212> PRT
<213> Homo sapiens

<400> 3
Met Glu Ile Thr Arg Ser His Thr Thr Ala Leu Glu Cys Lys Thr Val
1               5                   10                  15

Ser Pro Asp Glu Ile Thr Thr Arg Pro Met Asp Lys Thr Val Val Phe
            20                  25                  30
Val Asp Asn His Val Glu Leu Glu Met Thr Glu Ser His Thr Val Phe
        35                  40                  45
Ile Asp Tyr Gln Glu Lys Glu Arg Thr Asp Arg Pro Asn Phe Glu Leu
    50                  55                  60
Ser Gln Arg Lys Ser Leu Gly Thr Pro Thr Val Ile Cys Thr Pro Thr
65                  70                  75                  80
Glu Glu Ser Val Phe Phe Pro Gly Asn Gly Glu Ser Asp Arg Leu Val
                85                  90                  95
Ala Asn Asp Ser Gln Leu Thr Pro Leu Glu Glu Trp Ser Asn Asn Arg
            100                 105                 110
Gly Pro Val Glu Val Ala Asp Asn Met Glu Leu Ser Lys Ser Ala Thr
        115                 120                 125
Cys Lys Asn Ile Lys Asp Val Gln Ser Pro Gly Phe Leu Asn Glu Pro
    130                 135                 140
Leu Ser Ser Lys Ser Gln Arg Arg Lys Ser Leu Lys Leu Lys Asn Asp
145                 150                 155                 160
Lys Thr Ile Val Phe Ser Glu Asn His Lys Asn Asp Met Asp Ile Thr
                165                 170                 175

Gln Ser Cys Met Val Glu Ile Asp Asn Glu Ser Ala Leu Glu Asp Lys
            180                 185                 190
Glu Asp Phe His Leu Ala Gly Ala Ser Lys Thr Ile Leu Tyr Ser Cys
        195                 200                 205
Gly Gln Asp Asp Met Glu Ile Thr Arg Ser His Thr Thr Ala Leu Glu
    210                 215                 220
Cys Lys Thr Leu Leu Pro Asn Glu Ile Ala Ile Arg Pro Met Asp Lys
225                 230                 235                 240
Thr Val Leu Phe Thr Asp Asn Tyr Ser Asp Leu Glu Val Thr Asp Ser
                245                 250                 255
His Thr Val Phe Ile Asp Cys Gln Ala Thr Glu Lys Ile Leu Glu Glu
            260                 265                 270
Asn Pro Lys Phe Gly Ile Gly Lys Gly Lys Asn Leu Gly Val Ser Phe
        275                 280                 285
Pro Lys Asp Asn Ser Cys Val Gln Glu Ile Ala Glu Lys Gln Ala Leu
    290                 295                 300
Ala Val Gly Asn Lys Ile Val Leu His Thr Glu Gln Lys Gln Gln Leu
305                 310                 315                 320
Phe Ala Ala Thr Asn Arg Thr Thr Asn Glu Ile Ile Lys Phe His Ser
                325                 330                 335

Ala Ala Met Asp Glu Lys Val Ile Gly Lys Val Val Asp Gln Ala Cys
            340                 345                 350
Thr Leu Glu Lys Ala Gln Val Glu Ser Cys Gln Leu Asn Asn Arg Asp
        355                 360                 365
Arg Arg Asn Val Asp Phe Thr Ser Ser His Ala Thr Ala Val Cys Gly
    370                 375                 380
Ser Ser Asp Asn Tyr Ser Cys Leu Ala Asn Val Ile Ser Cys Thr Asp
385                 390                 395                 400
Asn Leu Glu Gly Ser Ala Met Leu Leu Cys Asp Lys Asp Glu Glu Lys
                405                 410                 415
Ala His Tyr Cys Pro Val Gln Asn Asp Leu Ala Tyr Ala Asn Asp Phe
            420                 425                 430
Ala Ser Glu Tyr Tyr Leu Glu Ser Glu Gly Gln Pro Leu Ser Ala Pro
        435                 440                 445
Cys Pro Leu Leu Glu Lys Glu Glu Val Ile Gln Thr Ser Thr Lys Gly
    450                 455                 460
Gln Leu Asp Cys Val Ile Thr Leu His Lys Asp Gln Asp Leu Ile Lys
465                 470                 475                 480
Asp Pro Arg Asn Leu Leu Ala Asn Gln Thr Leu Val Tyr Ser Gln Asp
                485                 490                 495

Leu Gly Glu Met Thr Lys Leu Asn Ser Lys Arg Val Ser Phe Lys Leu
            500                 505                 510
Pro Lys Asp Gln Met Lys Val Tyr Val Asp Asp Ile Tyr Val Ile Pro
        515                 520                 525
Gln Pro His Phe Ser Thr Asp Gln Pro Pro Leu Pro Lys Lys Gly Gln
    530                 535                 540
Ser Ser Ile Asn Lys Glu Glu Val Ile Leu Ser Lys Ala Gly Asn Lys
545                 550                 555                 560
Ser Leu Asn Ile Ile Glu Asn Ser Ser Ala Pro Ile Cys Glu Asn Lys
                565                 570                 575
Pro Lys Ile Leu Asn Ser Glu Glu Trp Phe Ala Ala Ala Cys Lys Lys
            580                 585                 590
Glu Leu Lys Glu Asn Ile Gln Thr Thr Asn Tyr Asn Thr Ala Leu Asp
        595                 600                 605
Phe His Ser Asn Ser Asp Val Thr Lys Gln Val Ile Gln Thr His Val
    610                 615                 620
Asn Ala Gly Glu Ala Pro Asp Pro Val Ile Thr Ser Asn Val Pro Cys
625                 630                 635                 640
Phe His Ser Ile Lys Pro Asn Leu Asn Asn Leu Asn Gly Lys Thr Gly
                645                 650                 655

Glu Phe Leu Ala Phe Gln Thr Val His Leu Pro Pro Leu Pro Glu Gln
            660                 665                 670
Leu Leu Glu Leu Gly Asn Lys Ala His Asn Asp Met His Ile Val Gln
        675                 680                 685
Ala Thr Glu Ile His Asn Ile Asn Ile Ile Ser Ser Asn Ala Lys Asp
    690                 695                 700
Ser Arg Asp Glu Glu Asn Lys Lys Ser His Asn Gly Ala Glu Thr Thr
705                 710                 715                 720
Ser Leu Pro Pro Lys Thr Val Phe Lys Asp Lys Val Arg Arg Cys Ser
                725                 730                 735
Leu Gly Ile Phe Leu Pro Arg Leu Pro Asn Lys Arg Asn Cys Ser Val
            740                 745                 750
Thr Gly Ile Asp Asp Leu Glu Gln Ile Pro Ala Asp Thr Thr Asp Ile
        755                 760                 765
Asn His Leu Glu Thr Gln Pro Val Ser Ser Lys Asp Ser Gly Ile Gly
    770                 775                 780
Ser Val Ala Gly Lys Leu Asn Leu Ser Pro Ser Gln Tyr Ile Asn Glu
785                 790                 795                 800
Glu Asn Leu Pro Val Tyr Pro Asp Glu Ile Asn Ser Ser Asp Ser Ile
                805                 810                 815

Tyr Ile Glu Thr Glu Glu Lys Ala Val Gly Thr Arg Arg Arg Arg Tyr
            820                 825                 830

Ser

<210> 4
<211> 9
<212> PRT
<213> Homo sapiens

<400> 4
Leu Leu Asp Asn Pro Ile Ser Glu Lys
 1               5

<210> 5
<211> 10
<212> PRT
<213> Homo sapiens

<400> 5
Gly Leu Leu Asp Asn Pro Ile Ser Glu Lys
 1               5                   10

<210> 6
<211> 9

<212> PRT
<213> Homo sapiens

<400> 6
Asp Val Thr Thr Gly Tyr Gly Thr Lys
1               5

<210> 7
<211> 9
<212> PRT
<213> Homo sapiens

<400> 7
Ser Val Gly Gly Pro Lys Ile Asp Lys
1               5

<210> 8
<211> 10
<212> PRT
<213> Homo sapiens

<400> 8
Asp Val Leu Glu Ala Glu Ser Val Gln Lys
1               5                   10

<210> 9
<211> 10

<212> PRT
<213> Homo sapiens

<400> 9
Gly Val Leu Asp Lys Gln Ile Thr Asn Arg
1               5                   10

<210> 10
<211> 9
<212> PRT
<213> Homo sapiens

<400> 10
His Thr Glu Asp Ser Arg Met Lys Lys
1               5

<210> 11
<211> 9
<212> PRT
<213> Homo sapiens

<400> 11
Lys Ile Asp Phe Asn Asp Phe Ile Lys
1               5

<210> 12
<211> 9

<212> PRT
<213> Homo sapiens

<400> 12
Thr Ile Glu Ile Asn His Arg Leu Tyr
1               5

<210> 13
<211> 9
<212> PRT
<213> Homo sapiens

<400> 13
Val Leu Glu Asp Glu Ser Val Gln Lys
1               5

<210> 14
<211> 9
<212> PRT
<213> Homo sapiens

<400> 14
Thr Ser Glu Ser His Ser Gln Ser Lys
1               5

<210> 15
<211> 9

<212> PRT
<213> Homo sapiens

<400> 15
Leu Thr Asp Thr Trp Asn Lys Asp Lys
1               5

<210> 16
<211> 9
<212> PRT
<213> Homo sapiens

<400> 16
Ile Ile Glu His Thr Arg Glu Arg Lys
1               5

<210> 17
<211> 9
<212> PRT
<213> Homo sapiens

<400> 17
Pro Ser Glu Leu Gln Glu Leu Gly Lys
1               5

<210> 18
<211> 9

<212> PRT
<213> Homo sapiens

<400> 18
Cys Asn Asp Ala Met Glu Met Thr Lys
1               5

<210> 19
<211> 10
<212> PRT
<213> Homo sapiens

<400> 19
Gln Thr Glu Ser His Met Lys Ile Val Arg
1               5                   10

<210> 20
<211> 10
<212> PRT
<213> Homo sapiens

<400> 20
Ser Ser Asp Glu Cys Glu Glu Ile Thr Lys
1               5                   10

<210> 21
<211> 10

<212> PRT
<213> Homo sapiens

<400> 21
Ser Ser Glu Thr Asn Ser Arg Glu Ser Lys
1               5                   10

<210> 22
<211> 10
<212> PRT
<213> Homo sapiens

<400> 22
Lys Ile Asp Phe Asn Asp Phe Ile Lys Arg
1               5                   10

<210> 23
<211> 10
<212> PRT
<213> Homo sapiens

<400> 23
Ser Gly Glu Glu Asn Met Asp Ile Thr Lys
1               5                   10

<210> 24
<211> 10

<212> PRT
<213> Homo sapiens

<400> 24
Ala Asn Glu Glu Asn Asp Asn Ile Glu Arg
1               5                   10

<210> 25
<211> 10
<212> PRT
<213> Homo sapiens

<400> 25
Glu Thr Glu Ala Gly Glu Asn Leu Leu Leu
1               5                   10

<210> 26
<211> 9
<212> PRT
<213> Homo sapiens

<400> 26
Ile Tyr Gly Asn Asp Phe Met Asp Leu
1               5

<210> 27
<211> 9

<212> PRT
<213> Homo sapiens

<400> 27
Asp Tyr Cys His Asp Lys Met Ile Ile
1               5

<210> 28
<211> 9
<212> PRT
<213> Homo sapiens

<400> 28
Asp Phe Asn Asp Phe Ile Lys Arg Leu
1               5

<210> 29
<211> 9
<212> PRT
<213> Homo sapiens

<400> 29
Thr Phe Asn His Thr Leu Gln Ile Leu
1               5

<210> 30
<211> 9

<212> PRT
<213> Homo sapiens

<400> 30
Phe Tyr Ser Ser Cys Asn Asp Ala Met
1               5

<210> 31
<211> 9
<212> PRT
<213> Homo sapiens

<400> 31
Lys Gln Asn Thr Ala Phe Gln Asp Leu
1               5

<210> 32
<211> 9
<212> PRT
<213> Homo sapiens

<400> 32
Lys Ser Asn Cys Ile Met Asp Val Leu
1               5

<210> 33
<211> 9

<212> PRT
<213> Homo sapiens

<400> 33
Ser Phe Ala Asp Thr Ile Lys Val Phe
1               5

<210> 34
<211> 9
<212> PRT
<213> Homo sapiens

<400> 34
Tyr Thr Ile Glu Ile Asn His Arg Leu
1               5

<210> 35
<211> 9
<212> PRT
<213> Homo sapiens

<400> 35
Leu Phe Ser Thr Thr Lys Pro Leu Phe
1               5

<210> 36
<211> 10

<212> PRT
<213> Homo sapiens

<400> 36
Ser Tyr Thr Ile Glu Ile Asn His Arg Leu
1               5                   10

<210> 37
<211> 10
<212> PRT
<213> Homo sapiens

<400> 37
Ile Tyr Ser Gly Glu Glu Asn Met Asp Ile
1               5                   10

<210> 38
<211> 10
<212> PRT
<213> Homo sapiens

<400> 38
Gly Phe Gly Pro Ser Glu Leu Gln Glu Leu
1               5                   10

<210> 39
<211> 10

<212> PRT
<213> Homo sapiens

<400> 39
Ile Phe Ser Asp Glu Asn Gln Met Asp Leu
1               5                   10

<210> 40
<211> 10
<212> PRT
<213> Homo sapiens

<400> 40
Arg Ile Gln Gln Ser Leu Ser Asn Pro Leu
1               5                   10

<210> 41
<211> 10
<212> PRT
<213> Homo sapiens

<400> 41
Lys Pro Pro Arg Ser Pro Leu Gln Asp Leu
1               5                   10

<210> 42
<211> 10

<212> PRT
<213> Homo sapiens

<400> 42
Lys Ser Val Leu Gly Gln Asn Ser Lys Leu
1               5                   10

<210> 43
<211> 10
<212> PRT
<213> Homo sapiens

<400> 43
Lys Ser Pro Ile Glu Lys Ser Gly Val Leu
1               5                   10

<210> 44
<211> 10
<212> PRT
<213> Homo sapiens

<400> 44
Lys Leu Ala Glu Pro Leu Arg Lys Ser Leu
1               5                   10

<210> 45
<211> 10

<212> PRT
<213> Homo sapiens

<400> 45
Val Phe Lys Ser Lys Gln Asn Thr Ala Phe
1               5                   10

<210> 46
<211> 9
<212> PRT
<213> Homo sapiens

<400> 46
Trp Val Leu Lys Ile Leu Pro Tyr Leu
1               5

<210> 47
<211> 9
<212> PRT
<213> Homo sapiens

<400> 47
Lys Met Asn Val Asn Cys Asn Ser Val
1               5

<210> 48
<211> 9

<212> PRT
<213> Homo sapiens

<400> 48
Leu Leu Leu Ile Gln Asn Lys Lys Leu
1               5

<210> 49
<211> 9
<212> PRT
<213> Homo sapiens

<400> 49
Ser Leu Thr Asp Arg Lys Thr Glu Leu
1               5

<210> 50
<211> 9
<212> PRT
<213> Homo sapiens

<400> 50
Ser Leu Phe Ser Thr Thr Lys Pro Leu
1               5

<210> 51
<211> 9

<212> PRT
<213> Homo sapiens

<400> 51
Met Met Leu Gln Asn Leu Met Thr Thr
1               5

<210> 52
<211> 9
<212> PRT
<213> Homo sapiens

<400> 52
Gln Met Gln Gln Lys Glu Phe Ser Ile
1               5

<210> 53
<211> 9
<212> PRT
<213> Homo sapiens

<400> 53
Leu Leu Ser Gly Glu Asn Thr Asp Leu
1               5

<210> 54
<211> 9

<212> PRT
<213> Homo sapiens

<400> 54
Ile Met Gly Ala Glu Thr His Ile Val
1               5

<210> 55
<211> 9
<212> PRT
<213> Homo sapiens

<400> 55
Phe Leu Ala Asn Leu Lys Leu His Thr
1               5

<210> 56
<211> 9
<212> PRT
<213> Homo sapiens

<400> 56
Thr Leu Leu Ser Ala Pro Ile His Thr
1               5

<210> 57
<211> 9

<212> PRT
<213> Homo sapiens

<400> 57
Lys Gln Ile Thr Asn Arg Asn Thr Val
1               5

<210> 58
<211> 9
<212> PRT
<213> Homo sapiens

<400> 58
Phe Gln Thr Glu Ser His Met Lys Ile
1               5

<210> 59
<211> 9
<212> PRT
<213> Homo sapiens

<400> 59
Lys Met Tyr Cys Asn Pro Asp Ala Met
1               5

<210> 60
<211> 9

<212> PRT
<213> Homo sapiens

<400> 60
Phe Met Asp Leu Thr Phe Asn His Thr
1               5

<210> 61
<211> 9
<212> PRT
<213> Homo sapiens

<400> 61
Lys Leu His Thr Glu Asp Ser Arg Met
1               5

<210> 62
<211> 9
<212> PRT
<213> Homo sapiens

<400> 62
Lys Leu Glu Asp Asn Tyr Cys Glu Ile
1               5

<210> 63
<211> 9

<212> PRT
<213> Homo sapiens

<400> 63
Asn Leu Gly Ser Gln Val Pro Leu Ala
1               5

<210> 64
<211> 10
<212> PRT
<213> Homo sapiens

<400> 64
Phe Gln Thr Glu Ser His Met Lys Ile Val
1               5                   10

<210> 65
<211> 10
<212> PRT
<213> Homo sapiens

<400> 65
Phe Met Asp Leu Thr Phe Asn His Thr Leu
1               5                   10

<210> 66
<211> 10

<212> PRT
<213> Homo sapiens

<400> 66
Asn Leu Leu Leu Ile Gln Asn Lys Lys Leu
1               5                   10

<210> 67
<211> 10
<212> PRT
<213> Homo sapiens

<400> 67
Thr Met Asn Arg Gln Ile Ala Val Lys Val
1               5                   10

<210> 68
<211> 10
<212> PRT
<213> Homo sapiens

<400> 68
Ser Leu Thr Asp Arg Lys Thr Glu Leu Leu
1               5                   10

<210> 69
<211> 10

<212> PRT
<213> Homo sapiens

<400> 69
Val Ser Trp Glu Gln Ser Leu Phe Ser Thr
1               5                   10

<210> 70
<211> 10
<212> PRT
<213> Homo sapiens

<400> 70
Gln Ile Phe Lys Gln Asp Gln Ser Asn Val
1               5                   10

<210> 71
<211> 10
<212> PRT
<213> Homo sapiens

<400> 71
Thr Gln Met Gln Gln Lys Glu Phe Ser Ile
1               5                   10

<210> 72
<211> 10

<212> PRT
<213> Homo sapiens

<400> 72
Ala Ile Ser Ser His Thr Val Lys Ser Val
1               5                   10

<210> 73
<211> 10
<212> PRT
<213> Homo sapiens

<400> 73
Ser Leu Ser Asn Pro Leu Ser Ile Ser Leu
1               5                   10

<210> 74
<211> 10
<212> PRT
<213> Homo sapiens

<400> 74
Asn Met Asp Leu Thr Lys Ser His Thr Val
1               5                   10

<210> 75
<211> 10

<212> PRT
<213> Homo sapiens

<400> 75
Asn Met Asp Ile Thr Lys Ser His Thr Val
1               5                   10

<210> 76
<211> 10
<212> PRT
<213> Homo sapiens

<400> 76
Gln Met Asp Leu Thr Ser Ser His Thr Val
1               5                   10

<210> 77
<211> 10
<212> PRT
<213> Homo sapiens

<400> 77
Thr Ile Tyr Gly Asn Asp Phe Met Asp Leu
1               5                   10

<210> 78
<211> 10

<212> PRT
<213> Homo sapiens

<400> 78
Ile Met Asp Val Leu Glu Asp Glu Ser Val
1               5                   10

<210> 79
<211> 10
<212> PRT
<213> Homo sapiens

<400> 79
Asn Val Asn Cys Asn Ser Val Pro His Val
1               5                   10

<210> 80
<211> 10
<212> PRT
<213> Homo sapiens

<400> 80
Ser Leu Ser Asn Pro Thr Pro Asp Tyr Cys
1               5                   10

<210> 81
<211> 10

<212> PRT
<213> Homo sapiens

<400> 81
Asn Gln Met Asp Leu Thr Ser Ser His Thr
1               5                   10

<210> 82
<211> 10
<212> PRT
<213> Homo sapiens

<400> 82
Ser Ile Leu Lys Pro Pro Arg Ser Pro Leu
1               5                   10

<210> 83
<211> 9
<212> PRT
<213> Homo sapiens

<400> 83
Thr Val Met Ile Thr Lys Gly Leu Leu
1               5

<210> 84
<211> 9

<212> PRT
<213> Homo sapiens

<400> 84
Ser Val Leu Gly Gln Asn Ser Lys Leu
1               5

<210> 85
<211> 9
<212> PRT
<213> Homo sapiens

<400> 85
Gln Val Pro Leu Ala Ala Tyr Asn Leu
1               5

<210> 86
<211> 9
<212> PRT
<213> Homo sapiens

<400> 86
Val Ile Gly Phe Gly Pro Ser Glu Leu
1               5

<210> 87
<211> 10

<212> PRT
<213> Homo sapiens

<400> 87
Val Val Ile Gly Phe Gly Pro Ser Glu Leu
1               5                   10

<210> 88
<211> 9
<212> PRT
<213> Homo sapiens

<400> 88
Pro Pro Arg Ser Pro Leu Gln Asp Leu
1               5

<210> 89
<211> 10
<212> PRT
<213> Homo sapiens

<400> 89
Ala Pro Thr Pro Glu Lys Glu Met Met Leu
1               5                   10

<210> 90
<211> 9

<212> PRT
<213> Homo sapiens

<400> 90
Trp Asn Lys Asp Lys Asp Trp Val Leu
1               5

<210> 91
<211> 10
<212> PRT
<213> Homo sapiens

<400> 91
Asn Ser Arg Glu Ser Lys Gly Asn Asp Ile
1               5                   10

<210> 92
<211> 9
<212> PRT
<213> Homo sapiens

<400> 92
Glu Arg Pro Val Arg Arg Arg His Ser
1               5

<210> 93
<211> 10

<212> PRT
<213> Homo sapiens

<400> 93
Glu Arg Pro Val Arg Arg Arg His Ser Ser
1               5                   10

<210> 94
<211> 9
<212> PRT
<213> Homo sapiens

<400> 94
Cys Glu Ile Thr Gly Met Asn Thr Leu
1               5

<210> 95
<211> 10
<212> PRT
<213> Homo sapiens

<400> 95
Ser Glu Ile Glu Asn Gln Thr Gln Asn Ala
1               5                   10

<210> 96
<211> 10

<212> PRT
<213> Homo sapiens

<400> 96
Cys Glu Ile Thr Gly Met Asn Thr Leu Leu
1               5                   10

<210> 97
<211> 9
<212> PRT
<213> Homo sapiens

<400> 97
Ile Gln Gly Cys Lys Thr Val Phe Tyr
1               5

<210> 98
<211> 9
<212> PRT
<213> Homo sapiens

<400> 98
Ser Leu Ser Asn Pro Thr Pro Asp Tyr
1               5

<210> 99
<211> 9

<212> PRT

<213> Homo sapiens

<400> 99

Asn Leu Leu Lys His Asp Ser Asn Tyr

1               5

<210> 100

<211> 9

<212> PRT

<213> Homo sapiens

<400> 100

Asp Leu Arg Gly Gly Asn Glu Thr Val

1               5

<210> 101

<211> 9

<212> PRT

<213> Homo sapiens

<400> 101

Gln Ile Leu Pro Ala Thr Gly Asn Phe

1               5

<210> 102

<211> 9

<212> PRT
<213> Homo sapiens

<400> 102
Ile Gln Gln Ser Leu Ser Asn Pro Leu
1               5

<210> 103
<211> 9
<212> PRT
<213> Homo sapiens

<400> 103
Asp Ile Thr Ile Tyr Gly Asn Asp Phe
1               5

<210> 104
<211> 10
<212> PRT
<213> Homo sapiens

<400> 104
Arg Leu Lys Thr Gly Lys Cys Ser Ala Phe
1               5                   10

<210> 105
<211> 10

<212> PRT
<213> Homo sapiens

<400> 105
Ile Gln Asn Lys Lys Leu Glu Asp Asn Tyr
1               5                   10

<210> 106
<211> 10
<212> PRT
<213> Homo sapiens

<400> 106
Leu Gln Ile Leu Pro Ala Thr Gly Asn Phe
1               5                   10

<210> 107
<211> 10
<212> PRT
<213> Homo sapiens

<400> 107
Ile Leu Ala Met Thr Pro Glu Ser Ile Tyr
1               5                   10

<210> 108
<211> 10

<212> PRT
<213> Homo sapiens

<400> 108
Ser Leu Phe Ser Thr Thr Lys Pro Leu Phe
1               5                   10

<210> 109
<211> 10
<212> PRT
<213> Homo sapiens

<400> 109
Ser Ile Gln Gly Cys Lys Thr Val Phe Tyr
1               5                   10

<210> 110
<211> 10
<212> PRT
<213> Homo sapiens

<400> 110
Glu Ile Ala Thr Ser His Asn Ile Val Tyr
1               5                   10

<210> 111
<211> 10

<212> PRT
<213> Homo sapiens

<400> 111
Glu Ile Thr Lys Ser Arg Asn Glu Pro Phe
1               5                   10

<210> 112
<211> 9
<212> PRT
<213> Homo sapiens

<400> 112
Ala Met Asp Glu Lys Val Ile Gly Lys
1               5

<210> 113
<211> 10
<212> PRT
<213> Homo sapiens

<400> 113
Lys Leu Met Ser Lys Arg Val Ser Phe Lys
1               5                   10

<210> 114
<211> 9

<212> PRT
<213> Homo sapiens

<400> 114
Thr Val Phe Lys Asp Lys Val Arg Arg
1               5

<210> 115
<211> 10
<212> PRT
<213> Homo sapiens

<400> 115
Glu Val Ala Asp Asn Met Glu Leu Ser Lys
1               5                   10

<210> 116
<211> 9
<212> PRT
<213> Homo sapiens

<400> 116
Ser Thr Asp Gln Pro Pro Leu Pro Lys
1               5

<210> 117
<211> 9

<212> PRT
<213> Homo sapiens

<400> 117
Val Ala Asp Asn Met Glu Leu Ser Lys
1               5

<210> 118
<211> 9
<212> PRT
<213> Homo sapiens

<400> 118
Ile Asn Glu Glu Asn Leu Pro Val Tyr
1               5

<210> 119
<211> 9
<212> PRT
<213> Homo sapiens

<400> 119
His Thr Glu Gln Lys Gln Gln Leu Phe
1               5

<210> 120
<211> 9

<212> PRT
<213> Homo sapiens

<400> 120
Ala Thr Glu Ile His Asn Ile Asn Ile
1               5

<210> 121
<211> 9
<212> PRT
<213> Homo sapiens

<400> 121
Phe Ile Asp Cys Gln Ala Thr Glu Lys
1               5

<210> 122
<211> 10
<212> PRT
<213> Homo sapiens

<400> 122
Ser Thr Asp Gln Pro Pro Leu Pro Lys Lys
1               5                   10

<210> 123
<211> 10

<212> PRT
<213> Homo sapiens

<400> 123
Gln Leu Asp Cys Val Ile Thr Leu His Lys
1               5                   10

<210> 124
<211> 10
<212> PRT
<213> Homo sapiens

<400> 124
Val Val Asp Gln Ala Cys Thr Leu Glu Lys
1               5                   10

<210> 125
<211> 10
<212> PRT
<213> Homo sapiens

<400> 125
Gly Ala Glu Thr Thr Ser Leu Pro Pro Lys
1               5                   10

<210> 126
<211> 10

<212> PRT
<213> Homo sapiens

<400> 126
Glu Thr Glu Glu Lys Ala Val Gly Thr Arg
1               5                   10

<210> 127
<211> 10
<212> PRT
<213> Homo sapiens

<400> 127
His Leu Glu Thr Gln Pro Val Ser Ser Lys
1               5                   10

<210> 128
<211> 10
<212> PRT
<213> Homo sapiens

<400> 128
Leu Ser Ala Pro Cys Pro Leu Leu Glu Lys
1               5                   10

<210> 129
<211> 10

<212> PRT
<213> Homo sapiens

<400> 129
His Thr Glu Gln Lys Gln Gln Leu Phe Ala
1               5                   10

<210> 130
<211> 9
<212> PRT
<213> Homo sapiens

<400> 130
His Tyr Cys Pro Val Gln Asn Asp Leu
1               5

<210> 131
<211> 9
<212> PRT
<213> Homo sapiens

<400> 131
Ile Tyr Val Ile Pro Gln Pro His Phe
1               5

<210> 132
<211> 9

<212> PRT
<213> Homo sapiens

<400> 132
Val Tyr Val Asp Asp Ile Tyr Val Ile
1               5

<210> 133
<211> 9
<212> PRT
<213> Homo sapiens

<400> 133
Asn Tyr Ser Cys Leu Ala Asn Val Ile
1               5

<210> 134
<211> 9
<212> PRT
<213> Homo sapiens

<400> 134
Val Phe Val Asp Asn His Val Glu Leu
1               5

<210> 135
<211> 9

<212> PRT
<213> Homo sapiens

<400> 135
Cys Phe His Ser Ile Lys Pro Asn Leu
1               5

<210> 136
<211> 9
<212> PRT
<213> Homo sapiens

<400> 136
Val Tyr Ser Gln Asp Leu Gly Glu Met
1               5

<210> 137
<211> 9
<212> PRT
<213> Homo sapiens

<400> 137
Leu Tyr Ser Cys Gly Gln Asp Asp Met
1               5

<210> 138
<211> 9

<212> PRT
<213> Homo sapiens

<400> 138
Leu Phe Thr Asp Asn Tyr Ser Asp Leu
1               5

<210> 139
<211> 9
<212> PRT
<213> Homo sapiens

<400> 139
His Phe Ser Thr Asp Gln Pro Pro Leu
1               5

<210> 140
<211> 9
<212> PRT
<213> Homo sapiens

<400> 140
Lys Val Val Asp Gln Ala Cys Thr Leu
1               5

<210> 141
<211> 9

<212> PRT
<213> Homo sapiens

<400> 141
Arg Asn Leu Leu Ala Asn Gln Thr Leu
1               5

<210> 142
<211> 9
<212> PRT
<213> Homo sapiens

<400> 142
Arg Leu Val Ala Asn Asp Ser Gln Leu
1               5

<210> 143
<211> 9
<212> PRT
<213> Homo sapiens

<400> 143
Lys Ala Gln Val Glu Ser Cys Gln Leu
1               5

<210> 144
<211> 9

<212> PRT
<213> Homo sapiens

<400> 144
Val Tyr Pro Asp Glu Ile Asn Ser Ser
1               5

<210> 145
<211> 10
<212> PRT
<213> Homo sapiens

<400> 145
Tyr Tyr Leu Glu Ser Glu Gly Gln Pro Leu
1               5                   10

<210> 146
<211> 10
<212> PRT
<213> Homo sapiens

<400> 146
Lys Phe Gly Ile Gly Lys Gly Lys Asn Leu
1               5                   10

<210> 147
<211> 10

<212> PRT
<213> Homo sapiens

<400> 147
Asn Phe Glu Leu Ser Gln Arg Lys Ser Leu
1               5                   10

<210> 148
<211> 10
<212> PRT
<213> Homo sapiens

<400> 148
Glu Phe Leu Ala Phe Gln Thr Val His Leu
1               5                   10

<210> 149
<211> 10
<212> PRT
<213> Homo sapiens

<400> 149
Ala Phe Gln Thr Val His Leu Pro Pro Leu
1               5                   10

<210> 150
<211> 10

<212> PRT
<213> Homo sapiens

<400> 150
Trp Phe Ala Ala Ala Cys Lys Lys Glu Leu
1               5                   10

<210> 151
<211> 10
<212> PRT
<213> Homo sapiens

<400> 151
Lys Ser Gln Arg Arg Lys Ser Leu Lys Leu
1               5                   10

<210> 152
<211> 10
<212> PRT
<213> Homo sapiens

<400> 152
Lys Gly Gln Leu Asp Cys Val Ile Thr Leu
1               5                   10

<210> 153
<211> 9

<212> PRT
<213> Homo sapiens

<400> 153
Lys Val Tyr Val Asp Asp Ile Tyr Val
1               5

<210> 154
<211> 9
<212> PRT
<213> Homo sapiens

<400> 154
Ile Leu Asn Ser Glu Glu Trp Phe Ala
1               5

<210> 155
<211> 9
<212> PRT
<213> Homo sapiens

<400> 155
Asn Leu Leu Ala Asn Gln Thr Leu Val
1               5

<210> 156
<211> 9

<212> PRT

<213> Homo sapiens

<400> 156

Lys Leu Pro Lys Asp Gln Met Lys Val
1               5

<210> 157

<211> 9

<212> PRT

<213> Homo sapiens

<400> 157

Ser Leu Gly Ile Phe Leu Pro Arg Leu
1               5

<210> 158

<211> 9

<212> PRT

<213> Homo sapiens

<400> 158

Tyr Ile Asn Glu Glu Asn Leu Pro Val
1               5

<210> 159

<211> 9

<212> PRT
<213> Homo sapiens

<400> 159
Phe Leu Ala Phe Gln Thr Val His Leu
1               5

<210> 160
<211> 9
<212> PRT
<213> Homo sapiens

<400> 160
Gly Gln Leu Asp Cys Val Ile Thr Leu
1               5

<210> 161
<211> 9
<212> PRT
<213> Homo sapiens

<400> 161
Gln Leu Leu Glu Leu Gly Asn Lys Ala
1               5

<210> 162
<211> 9

<212> PRT

<213> Homo sapiens

<400> 162

Thr Leu Leu Pro Asn Glu Ile Ala Ile

1               5

<210> 163

<211> 9

<212> PRT

<213> Homo sapiens

<400> 163

Ala Leu Ala Val Gly Asn Lys Ile Val

1               5

<210> 164

<211> 9

<212> PRT

<213> Homo sapiens

<400> 164

Gln Leu Phe Ala Ala Thr Asn Arg Thr

1               5

<210> 165

<211> 9

<212> PRT
<213> Homo sapiens

<400> 165
Phe Gln Thr Val His Leu Pro Pro Leu
1               5

<210> 166
<211> 9
<212> PRT
<213> Homo sapiens

<400> 166
Cys Leu Ala Asn Val Ile Ser Cys Thr
1               5

<210> 167
<211> 9
<212> PRT
<213> Homo sapiens

<400> 167
Val Ile Pro Gln Pro His Phe Ser Thr
1               5

<210> 168
<211> 10

<212> PRT
<213> Homo sapiens

<400> 168
Lys Ile Leu Asn Ser Glu Glu Trp Phe Ala
1               5                   10

<210> 169
<211> 10
<212> PRT
<213> Homo sapiens

<400> 169
Val Leu Phe Thr Asp Asn Tyr Ser Asp Leu
1               5                   10

<210> 170
<211> 10
<212> PRT
<213> Homo sapiens

<400> 170
Lys Leu Asn Leu Ser Pro Ser Gln Tyr Ile
1               5                   10

<210> 171
<211> 10

<212> PRT
<213> Homo sapiens

<400> 171
Arg Pro Met Asp Lys Thr Val Val Phe Val
1               5                   10

<210> 172
<211> 10
<212> PRT
<213> Homo sapiens

<400> 172
Ile Leu Asn Ser Glu Glu Trp Phe Ala Ala
1               5                   10

<210> 173
<211> 10
<212> PRT
<213> Homo sapiens

<400> 173
Arg Leu Pro Asn Lys Arg Asn Cys Ser Val
1               5                   10

<210> 174
<211> 10

<212> PRT
<213> Homo sapiens

<400> 174
Asn Leu Asn Gly Lys Thr Gly Glu Phe Leu
1               5                   10

<210> 175
<211> 10
<212> PRT
<213> Homo sapiens

<400> 175
Ile Leu Ser Lys Ala Gly Asn Lys Ser Leu
1               5                   10

<210> 176
<211> 10
<212> PRT
<213> Homo sapiens

<400> 176
Val Leu His Thr Glu Gln Lys Gln Gln Leu
1               5                   10

<210> 177
<211> 10

<212> PRT
<213> Homo sapiens

<400> 177
Ala Leu Glu Asp Lys Glu Asp Phe His Leu
1               5                   10

<210> 178
<211> 10
<212> PRT
<213> Homo sapiens

<400> 178
Ala Leu Asp Phe His Ser Asn Ser Asp Val
1               5                   10

<210> 179
<211> 10
<212> PRT
<213> Homo sapiens

<400> 179
Ile Leu Glu Glu Asn Pro Lys Phe Gly Ile
1               5                   10

<210> 180
<211> 10

<212> PRT
<213> Homo sapiens

<400> 180
Ala Met Asp Glu Lys Val Ile Gly Lys Val
1               5                   10

<210> 181
<211> 10
<212> PRT
<213> Homo sapiens

<400> 181
Tyr Val Ile Pro Gln Pro His Phe Ser Thr
1               5                   10

<210> 182
<211> 10
<212> PRT
<213> Homo sapiens

<400> 182
Ser Leu Gly Thr Pro Thr Val Ile Cys Thr
1               5                   10

<210> 183
<211> 10

<212> PRT
<213> Homo sapiens

<400> 183
Leu Leu Cys Asp Lys Asp Glu Glu Lys Ala
1               5                   10

<210> 184
<211> 10
<212> PRT
<213> Homo sapiens

<400> 184
Gln Leu Asn Asn Arg Asp Arg Arg Asn Val
1               5                   10

<210> 185
<211> 10
<212> PRT
<213> Homo sapiens

<400> 185
Ile Leu Tyr Ser Cys Gly Gln Asp Asp Met
1               5                   10

<210> 186
<211> 10

<212> PRT
<213> Homo sapiens

<400> 186
Glu Met Thr Glu Ser His Thr Val Phe Ile
1               5                   10

<210> 187
<211> 10
<212> PRT
<213> Homo sapiens

<400> 187
Val Val Phe Val Asp Asn His Val Glu Leu
1               5                   10

<210> 188
<211> 10
<212> PRT
<213> Homo sapiens

<400> 188
Lys Val Tyr Val Asp Asp Ile Tyr Val Ile
1               5                   10

<210> 189
<211> 10

<212> PRT

<213> Homo sapiens

<400> 189

Val Ile Gln Thr Ser Thr Lys Gly Gln Leu
1               5                   10

<210> 190

<211> 10

<212> PRT

<213> Homo sapiens

<400> 190

Val Ile Thr Leu His Lys Asp Gln Asp Leu
1               5                   10

<210> 191

<211> 10

<212> PRT

<213> Homo sapiens

<400> 191

Ile Gln Thr Thr Asn Tyr Asn Thr Ala Leu
1               5                   10

<210> 192

<211> 10

<212> PRT
<213> Homo sapiens

<400> 192
Leu Val Tyr Ser Gln Asp Leu Gly Glu Met
1               5                   10

<210> 193
<211> 9
<212> PRT
<213> Homo sapiens

<400> 193
Leu Pro Pro Leu Pro Glu Gln Leu Leu
1               5

<210> 194
<211> 10
<212> PRT
<213> Homo sapiens

<400> 194
Gln Pro Leu Ser Ala Pro Cys Pro Leu Leu
1               5                   10

<210> 195
<211> 9

<212> PRT
<213> Homo sapiens

<400> 195
Ser Gln Arg Arg Lys Ser Leu Lys Leu
1               5

<210> 196
<211> 10
<212> PRT
<213> Homo sapiens

<400> 196
Ser Ser Lys Ser Gln Arg Arg Lys Ser Leu
1               5                   10

<210> 197
<211> 9
<212> PRT
<213> Homo sapiens

<400> 197
Ser Lys Ser Gln Arg Arg Lys Ser Leu
1               5

<210> 198
<211> 10

<212> PRT
<213> Homo sapiens

<400> 198
Asp Arg Leu Val Ala Asn Asp Ser Gln Leu
1               5                   10

<210> 199
<211> 9
<212> PRT
<213> Homo sapiens

<400> 199
Val Glu Val Ala Asp Asn Met Glu Leu
1               5

<210> 200
<211> 9
<212> PRT
<213> Homo sapiens

<400> 200
Val Glu Ile Asp Asn Glu Ser Ala Leu
1               5

<210> 201
<211> 9

<212> PRT
<213> Homo sapiens

<400> 201
Gln Glu Ile Ala Glu Lys Gln Ala Leu
1               5

<210> 202
<211> 10
<212> PRT
<213> Homo sapiens

<400> 202
Met Glu Ile Thr Arg Ser His Thr Thr Ala
1               5                   10

<210> 203
<211> 10
<212> PRT
<213> Homo sapiens

<400> 203
Gln Glu Ile Ala Glu Lys Gln Ala Leu Ala
1               5                   10

<210> 204
<211> 10

<212> PRT
<213> Homo sapiens

<400> 204
Asn Glu Ile Ile Lys Phe His Ser Ala Ala
1               5                   10

<210> 205
<211> 9
<212> PRT
<213> Homo sapiens

<400> 205
Leu Leu Ala Asn Gln Thr Leu Val Tyr
1               5

<210> 206
<211> 9
<212> PRT
<213> Homo sapiens

<400> 206
Asn Leu Asn Gly Lys Thr Gly Glu Phe
1               5

<210> 207
<211> 9

<212> PRT
<213> Homo sapiens

<400> 207
Lys Leu Asn Leu Ser Pro Ser Gln Tyr
1               5

<210> 208
<211> 9
<212> PRT
<213> Homo sapiens

<400> 208
Lys Leu Asn Ser Lys Arg Val Ser Phe
1               5

<210> 209
<211> 9
<212> PRT
<213> Homo sapiens

<400> 209
Lys Gly Lys Asn Leu Gly Val Ser Phe
1               5

<210> 210
<211> 9

<212> PRT
<213> Homo sapiens

<400> 210
Ser Gln Leu Thr Pro Leu Glu Glu Trp
1               5

<210> 211
<211> 9
<212> PRT
<213> Homo sapiens

<400> 211
Glu Gln Lys Gln Gln Leu Phe Ala Ala
1               5

<210> 212
<211> 9
<212> PRT
<213> Homo sapiens

<400> 212
Lys Ile Leu Glu Glu Asn Pro Lys Phe
1               5

<210> 213
<211> 9

<212> PRT

<213> Homo sapiens

<400> 213

Val Ile Thr Ser Asn Val Pro Cys Phe

1               5

<210> 214

<211> 10

<212> PRT

<213> Homo sapiens

<400> 214

Lys Leu Lys Asn Asp Lys Thr Ile Val Phe

1               5                   10

<210> 215

<211> 10

<212> PRT

<213> Homo sapiens

<400> 215

Asn Leu Leu Ala Asn Gln Thr Leu Val Tyr

1               5                   10

<210> 216

<211> 10

<212> PRT
<213> Homo sapiens

<400> 216
Lys Leu Pro Lys Asp Gln Met Lys Val Tyr
1               5                   10

<210> 217
<211> 10
<212> PRT
<213> Homo sapiens

<400> 217
Asn Ile Lys Asp Val Gln Ser Pro Gly Phe
1               5                   10

<210> 218
<211> 10
<212> PRT
<213> Homo sapiens

<400> 218
Ser Gln Arg Lys Ser Leu Gly Thr Pro Thr
1               5                   10

<210> 219
<211> 10

<212> PRT

<213> Homo sapiens

<400> 219

Glu Gln Lys Gln Gln Leu Phe Ala Ala Thr
1               5                   10

<210> 220

<211> 10

<212> PRT

<213> Homo sapiens

<400> 220

Tyr Ile Asn Glu Glu Asn Leu Pro Val Tyr
1               5                   10

<210> 221

<211> 10

<212> PRT

<213> Homo sapiens

<400> 221

Asp Ile Tyr Val Ile Pro Gln Pro His Phe
1               5                   10

<210> 222

<211> 22

<212> DNA
<213> Artificial Sequence

<220>
<223> Description of Artificial Sequence:Primer MT194

<400> 222
gagtcctcgg tgtgaagctt ta                                                22

<210> 223
<211> 22
<212> DNA
<213> Artificial Sequence

<220>
<223> Description of Artificial Sequence:Primer MT195

<400> 223
acagtgtgac ttgatgtcag gt                                                22

<210> 224
<211> 17
<212> DNA
<213> Artificial Sequence

<220>
<223> Description of Artificial Sequence:Forward Primer
      MT1149

<400> 224
cacatccagt gagacca                                                    17

<210> 225
<211> 17
<212> DNA
<213> Artificial Sequence

<220>
<223> Description of Artificial Sequence:Reverse Primer
      MT152

<400> 225
tcccatcttc tgatgtg                                                    17

<210> 226
<211> 18
<212> DNA
<213> Artificial Sequence

<220>
<223> Description of Artificial Sequence:Forward Primer
      MT227

<400> 226
caggaatcca atgcttgg                                                   18

<210> 227

<211> 17

<212> DNA

<213> Artificial Sequence

<220>

<223> Description of Artificial Sequence:Reverse Primer
      MT188

<400> 227
ctgttgcagg taagatc                                                17

## Description

### Technical Field

The present invention relates to: a cancer/testis antigen protein or peptide; a cancer/testis gene encoding the cancer/testis antigen protein or the peptide; a screening method for a promoter or a suppressor for the immunity-inducing activity using the cancer/testis antigen protein/peptide; an anti-tumor agent with the cancer/testis antigen protein or a partial peptide of the antigen protein or the like as an effective ingredient; a diagnostic probe for cancer using the cancer/testis gene; etc.

### Background Art

Tumor cells often express various genes such as a particular kind of protooncogenes which are not usually expressed in normal cells or at a considerably low level even if expressed (Science 235, 305-11, 1987). Gene populations expressed in such an abnormal manner include populations that are involved in tissue-specific differentiation (Immunol. Today 18, 267-8, 1997), and the expression of these genes in tumor cells are thought to be responsible for the malignant phenotype of the tumor cells. Many testis-specific genes are expressed in the testis (Reprod. Fertility & Develop. 7, 695-704, 1995, Int. J. Develop. Biol. 40, 379-83, 1996), however, most of these genes are reported not to be activated at all in ordinary tumors or very scarcely if any (Biochem. Biophys. Res. Comm. 241, 653-657, 1997). Recent studies, however, identified a population of genes which are expressed both in cancer and normal testis and some of which induce immune response in a host. These genes are called cancer-testis antigens (CT antigen). Besides, genes whose base sequences are homologous to cancer/testis antigen genes but which are expressed not only in testis but in genital organs of both sexes are also known (Proc. Natl. Acad. Sci. USA 95, 10757-62, 1998, Cancer Res. 59, 1445-8, 1999, J. Biol. Chem. 273, 17618-25, 1998).

The genes mentioned above including genes encoding cancer/testis antigens are called cancer/testis-related genes and are classified as shown in Table 1 below. As for cancer/testis-related genes encoding antigens which induce immune response in cancer patients, genes such as MAGE, BAGE, GAGE, LAGE, SSX, etc. are known (Science 254, 1643-7, 1991, Immunity 2, 167-75, 1995, J. Exp. Med. 182, 689-98, 1995, Int. J. Cancer 76, 903-8, 1998, Int. J. Cancer 72, 965-71, 1997, Cancer Res. 56, 4766-72, 1996). These antigens are identified according to their production of antibodies or responsiveness to cytotoxic T lymphocytes in cancer patients (Proc. Natl. Acad. Sci. USA 94, 1914-8, 1997, Science 254, 1643-7, 1991). On the other hand, known cancer/testis-related genes include some genes whose antigenicity is not yet elucidated (The Cancer J. from Scientific American, 16-7, 1999, Proc. Natl. Acad. Sci. USA 92, 11810-3, 1995, Cancer Res. 59, 3215-21, 1999. Proc. Natl. Acad. Sci. USA 95, 10757-62, 1998, Cancer Res. 59, 1445-8, 1999). There is a possibility that the in vivo expression of the former is frequent enough to raise immune response while the expression of the latter is not enough. Although original physiological functions of the cancer/testis-related genes largely remain unknown, the genes have been studied for analyzing of the specific gene expression mechanism in a tumor and the testis and for searching a possibility of application to diagnosis and the therapy for cancer (Cancer Res. 55, 3478-82, 1995, Int. J. Cancer 80, 219-30, 1999).

**Table 1**

| | |
|---|---|
| 1) Genes where their gene products have antigenicity (cancer/testis antigens). | MAGE, GAGE, BAGE, LAGE, SSX, SCP1 |
| 2) Genes where their gene products have no antigenicity | BRS-3, TSP-50 |
| 3) Genes having 00.sequence homology with cancer/testis antigens but being expressed in plural genitourinary organs other than the testis. | PAGE |

Most of the previously known cancer/testis-related genes exist in human X chromosomes. For instance, MAGE subfamily exists in four regions on X chromosome, i.e. Xq28, Xq21.3, Xq26 and Xq11.23 (Immunogenet. 40, 360-9, 1994, Cancer Res. 58, 743-52, 1998, Genomics 59, 161-7, 1999). Further, SSX genes exist on Xq11.2 (Nature Genet. 7, 502-8, 1994), LAGE1 and NY-ESO-1 genes exist on Xq28 (Cancer J From Scientific American 5: 16-17, 1999, Intl J Cancer 76, 903-908, 1998) and GAGE genes exist between Xp11.2-Xp11.4 (Cancer Res. 59, 3157-3165, 1999). Recently, however, the synaptonemal complex protein, which is known to exist on chromosome 1 (SCP1), has been shown as a member of the cancer/testis-related gene population (EMBO J. 11, 5091-5100, 1992), which, at the same time, has been elucidated to be identical with the HOM-TES-14 gene (Cytogen Cell Genet 78, 103-104, 1997, Proc Natl Acad Sci USA 95, 5211-5216, 1998).

On the other hand, rejection of a living body to cancer is known to be caused by cytotoxic T lymphocytes which have recognized the complex of a cancer antigen peptide and a histocompatibility antigen molecule (HLA) that are bound in a tissue or in a cell. Because a cancer antigen binds to HLA which is expressed in the tumor cell, it is known that a cytotoxic lymphocyte recognizes the complex as an antigen and thus causes rejection which results in the regression of a cancer cell. Marie Marchand et al. reported that they found a sequence comprising 9 amino acid residues in the MAGE 3 gene product (protein) has a property to bind to HLA-A1, one of HLA class I molecules, that they synthesized the peptide containing this amino acid sequence and immunized malignant melanoma patients with the peptide, and that tumor regression was observed in 7 out of 25 patients (Int. J. Cancer 80, 219-30, 1999).

Further, the transcription factor GCF is known as a factor which binds to the transcription regulatory region of a EGF (epidermal growth factor) receptor gene (Cell 59, 815-25, 1989). However, the previously reported GCFcDNA has recently been proved as being an artificial fusion molecule (Biochem. Biophys. Acta 1447, 125-31, 1999). That is to say, its sequence on the amino-terminal is derived from GCF2 which is a recently and newly identified transcription factor (J Biol. Chem. 273, 21594-602, 1998), and the most of the remaining cDNA is comprised of a part tentatively named GCF1 by the present inventors. It has been made clear that the base sequence-specific DNA-binding activity is found in GCF2, whereas GCF1 is devoid of the DNA-binding activity (Biochem. Biophys. Acta 1447, 125-31, 1999). However, it has not yet been known that a gene encoding a protein which specifically binds to GCF1 is a cancer/testis-related gene.

Other than the above, the yeast Two-hybrid System (Nature 340, 245-6, 1989, Proc. Natl. Acad. Sci. USA 88, 9578-82, 1991) is known as a system for detecting the in vivo protein-protein interactions using saccaromyces yeast. A usually used protein in the yeast Two-hybrid System is Gal4, a transcription activator for yeast which has a DNA-binding domain and a transcription-activating domain that can be separated from one another. For example, a plasmid vector carrying a gene encoding a fusion protein of the Gal4 DNA-binding domain and protein X, and a plasmid vector carrying a gene encoding a fusion protein of the Gal4 transcription-activating domain and protein Y are introduced into yeast containing a reporter gene such as LacZ gene, HIS3 gene, etc. and then the X-Y interaction can be detected by observing whether the function of Gal4 recovers and the transcription of a reporter gene is activated. When a known protein is used as X and cDNA library is used as a gene encoding Y in this yeast Two-hybrid System, a gene encoding a protein which interacts with X can be screened from the aforementioned cDNA library.

Cancer antigens are classified as in Table 2 based on the recent molecular biological studies. Since no class I molecule of a human histocompatibility antigen molecule (HLA) is expressed in the testis, a protein on a testis cell membrane is not recognized by a T lymphocyte. This gives us the view that a cancer/testis antigen is a cancer-specific antigen in a narrow sense because immunologically it is highly specific to cancer in spite of the fact that it is expressed in the testis. Since tissue-specific differentiated antigens are also expressed, though weakly, in normal tissues, adverse reaction on normal cells is causing drawbacks in performing cancer immunotherapy, and mutation-derived cancer antigens have drawbacks as well in that such mutation is limited only to the individual cancers, and therefore these antigens are hardly regarded as appropriate for the use in cancer immunotherapy. On the other hand, cancer/testis antigens are not expressed in normal tissues other than in the testis and are expressed in various cancers over a broad range, so that their use in immunotherapy agents for cancer is highly expected. The object of the present invention is to a provide a cancer/testis antigen applicable to diagnosis and immunotherapy for cancer, that is to say, a cancer/testis antigen which is not expressed in normal tissues other than the testis and which is expressed in various cancers over a broad range and induces immune response in a host, and a cancer/testis gene encoding the cancer/testis antigen and the like.

**Table 2**

| | |
|---|---|
| 1) Cancer specific antigen | Cancer/testis antigen |
| 2) Tissue specific differentiation antigen | tyrosinase, Melan/Mart-1, Pmel-17/gp100, TRP-1/gp75 |
| 3) Mutation-derived antigen | cdk4, caspase8 |
| 4) Overexpression-derived antigen | HER-2/neu, SART-1 |
| 5) Mutin | |
| 6) Viral antigen | E7 of HPV16 |

### Disclosure of the Invention

The present inventors have made a keen study to attain the objects mentioned above and by using the yeast Two-hybrid System, the present inventors screened a protein which interacts with GCF (GC element binding Factor), a previously reported transcription factor, as a gene controlling the proliferation of mammalian cells, cloned a gene for a protein which specifically binds to the transcription factor GCF, confirmed that the protein D40, the resulting gene product, is a cancer/testis antigen which is expressed substantially only in the testis in normal human tissues but is expressed in human primary cancers over a broad range deriving from various tissues and cells, and found that the D40 shows high affinity with the human histocompatibility antigen HLA and has a sequence comprising 9 or 10 amino acid residues which binds to a plurality of HLAs. Here, the present invention is completed.

The present invention relates to: a gene encoding the following protein (a) or (b), (a) a protein comprising the amino acid sequence shown by SEQ ID NO:2, (b) a protein which comprises an amino acid sequence wherein one or a few amino acids are deleted, substituted or added in the amino acid sequence shown by SEQ ID NO:2 and which has the immunity-inducing activity (claim 1); a gene encoding the following protein (a) or (b), (a) a protein comprising the amino acid sequence shown by SEQ ID NO:3, (b) a protein which comprises an amino acid sequence wherein one or a few amino acids are deleted, substituted or added in the amino acid sequence shown by SEQ ID NO:3 and which has the immunity-inducing activity (claim 2); DNA containing the base sequence shown by SEQ ID NO:1 or its complementary sequence and part of whole of these sequences (claim 3); and DNA which hybridizes under a stringent condition with DNA of claim 3 that forms a gene, and which encodes a protein having the immunity-inducing activity (claim 4).

The present invention further relates to: a protein comprising the amino acid sequence shown by SEQ ID NO:2 (claim 5); a protein which comprises an amino acid sequence wherein one or a few amino acids are deleted, substituted or added in the amino acid sequence shown by SEQ ID NO:2 and which has the immunity-inducing activity (claim 6); a protein comprising the amino acid sequence shown by SEQ ID NO:3 (claim 7); and a protein which comprises an amino acid sequence wherein one or a few amino acids are deleted, substituted or added in the amino acid sequence shown by SEQ ID NO:3 and which has the immunity-inducing activity (claim 8).

The present invention still further relate to: a peptide which comprises part of the protein of any of claims 5 to 8 and which binds to a histocompatibility antigen molecule Class I (claim 9); the peptide according to claim 9, wherein said peptide comprises part of the protein of claim 5 or 6 and binds to a histocompatibility antigen molecule Class I (claim 10); the peptide according to claim 10, wherein said peptide comprises an amino acid sequence shown by any of SEQ ID NOs:4-111 (claim 11); a peptide comprising the amino acid sequence of Ser-Tyr-Thr-Ile-Glu-Ile-Asn-His-Arg-Leu (claim 12); the peptide according to claim 9, wherein said peptide comprises part of the protein of claim 7 or 8 and binds to the histocompatibility antigen molecule Class I (claim 13); and the peptide according to claim 13, wherein said peptide comprises an amino acid sequence shown by any of SEQ ID NOs: 112-221 (claim 14).

The present invention also relates to: a fusion protein or a fusion peptide wherein the protein of claim 5 or 6 or the peptide of any of claims 10 to 12, and a marker protein and/or a peptide tag, are bound (claim 15); a fusion protein or a fusion peptide wherein the protein of claim 7 or 8 or the peptide of claim 13 or 14, and a marker protein and/or a peptide tag, are bound (claim 16); an antibody which specifically binds to the protein of claim 5 or 6 or to the peptide of any of claims 10 to 12 (claim 17); an antibody which specifically binds to the protein of claim 7 or 8 or to the peptide of claim 13 or 14 (claim 18); the antibody according to claim 17 or 18, wherein said antibody is a monoclonal antibody (claim 19); and a recombinant protein or peptide wherein the antibody of any of claims 17 to 19 specifically binds to said recombinant protein or peptide (claim 20).

The present invention further relates to: a host cell comprising an expression system capable of expressing the protein according to claim 5 or 6 (claim 21); a host cell comprising an expression system capable of expressing the protein according to claim 7 or 8 (claim 22); a non-human animal whose gene function to encode the protein of claim 5 or 6 is deficient on its chromosome (claim 23); a non-human animal whose gene function to encode the protein of claim 7 or 8 is deficient on its chromosome (claim 24); the non-human animal according to claim 23 or 24, wherein said non-human animal is a mouse or a rat (claim 25); a non-human animal which over-expresses the protein of claim 5 or 6 (claim 26); a non-human animal which over-expresses the protein of claim 7 or 8 (claim 27); and the non-human animal according to claim 26 or 27, wherein said non-human animal is a mouse or a rat (claim 28).

The present invention still further relates to: a method of screening a promoter or a suppressor for the immunity-inducing activity wherein the protein of claim 5 or 6, the peptide of any of claims 10 to 12 or a cell membrane expressing the protein of claim 5 or 6, and a test substance are used (claim 29); a method of screening a promoter or a suppressor for the immunity-inducing activity wherein the protein of claim 7 or 8, the peptide of claim 13 or 14, or a cell membrane expressing the protein of claim 7 or 8, and a test substance are used (claim 30); a method of screening a promoter or a suppressor for the immunity-inducing activity or for the expression of the protein of claim 5 or 6, wherein a cell expressing the protein and a test substance are used (claim 31); the method of screening a promoter or a suppressor for the immunity-inducing activity or for the expression of the protein of claim 5 or 6 according to claim 31, wherein the cell expressing the protein of claim 5 or 6 is the host cell of claim 21 (claim 32); a method of screening a promoter or a suppressor for the immunity-inducing activity or for the expression of the protein of claim 7 or 8, wherein a cell expressing the protein and a test substance are used (claim 33); the method of screening a promoter or a suppressor for the immunity-inducing activity or for the expression of the protein of claim 7 or 8 according to claim 33, wherein the cell expressing the protein of claim 7 or 8 is the host cell of claim 22 (claim 34); a method of screening a promoter or a suppressor for the immunity-inducing activity or a promoter or a suppressor for expression of the protein of any of claims 5 to 8, wherein the non-human animal of any of claims 23 to 25 and a test substance are used (claim 35); a method of screening a promoter or a suppressor for the immunity-inducing activity or a promoter or a suppressor for expression of the protein of any of claims 5 to 8, wherein the non-human animal of any of claims 26 to 28 and a test substance are used (claim 36); a promoter for the immunity-inducing activity obtained by the screening method according to any of claims 29 to 36 (claim 37); a suppressor for the immunity-inducing activity obtained by the screening method according to any of claims 29 to 36 (claim 38); an expression promoter for the protein of claim 5 or 6, wherein said expression promoter is obtained by the screening method according to any of claims 29 to 36 (claim 39); an expression suppressor for the protein of claim 7 or 8, wherein said expression suppressor is obtained by the screening method according to any of claims 29 to 36 (claim 40); an anti-tumor agent comprising the protein of any of claims 5 to 8, the peptide of any of claims 9 to 16, the protein or peptide of claim 20 or the antibody of any of claims 17 to 19, as an active ingredient (claim 41); and the anti-tumor agent according to claim 41, wherein the cancer is one or more cancers selected from uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma and colon cancer (claim 42).

The present invention further relates to: a method for detecting a cytotoxic T cell or its precursor cell wherein a HLA molecule, and the peptide of any of claims 9 to 16 or the peptide of claim 20 are used (claim 43); a method for detecting a cytotoxic T cell or its precursor cell wherein a complex of a HLA Class I molecule, and the peptide of any of claims 9 to 16 or the peptide of claim 20 is formed on the surface of fluorescent microparticles (claim 44); a detection reagent for a cytotoxic T cell or its precursor cell, wherein said detection reagent comprises a HLA molecule, and the peptide of any of claims 9 to 16 or the peptide of claim 20 (claim 45); a detection reagent for a cytotoxic T cell or its precursor cell wherein said detection reagent comprises: a complex of a HLA Class I molecule, and the peptide of any of claims 9 to 16 or the peptide of claim 20; and a fluorescent microparticle (claim 46); a cytotoxic T cell induced by in vitro stimuli wherein the protein of any of claims 5 to 8, the peptide of any of claims 9 to 16, or the protein or peptide of claim 20 is used (claim 47); a diagnostic probe for cancer comprising whole or part of the antisense strand of DNA or RNA encoding the protein of claim 5 or 6 (claim 48); a diagnostic probe for cancer comprising whole or part of the antisense strand of DNA or RNA encoding the protein of claim 7 or 8 (claim 49); a diagnostic drug for cancer comprising the diagnostic probe for cancer according to claim 48 or 49 and/or the antibody according to any of claims 17 to 19 (claim 50); and the diagnostic drug for cancer according to claim 50, wherein the cancer is one or more cancers selected from uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma and colon cancer (claim 51).

### Brief Description of Drawings

Fig. 1 is a drawing showing an expression site of D40 of the present invention in a normal human tissue.
Fig. 2 shows a part of the base sequence of D40cDNA and the amino acid sequence encoded by ORF which is encoded by the part of the base sequence.
Fig. 3 shows the analysis result of in vitro transcription and translation reactions of D40cDNA on SDS-PAGE using reticulocyte lysate.
Fig. 4 shows the result of Western blotting for in vivo transcription and translation reactions of D40cDNA using Cos7 cells.
Fig. 5 shows localization of D40 genes of the present invention on the chromosome by the in situ fluorescence hybridization.
Fig. 6 shows the expression result of D40mRNA in normal human tissues by Northern blot method.
Fig. 7 shows the expression result of D40mRNA in normal human tissues by RT-PCR method.
Fig. 8 shows the result of the cytotoxic activity caused by T cells from peripheral blood of cancer patients.

### Best Mode of Carrying Out the Invention

A cancer/testis-related gene of the present invention can be screened by a yeast Two-hybrid System as a gene encoding a protein which interacts with the transcription factor GCF, from a cDNA library or a genomic DNA library. Further, a cancer/testis antigen protein of the present invention can be obtained by expressing the cancer/testis-related gene obtained by such screening. Here, a cancer/testis-related gene is defined as follows: its expression is observed specifically in the normal testis and is not substantially observed in other normal tissues; it activates in a malignant tumor, as a result of which high level mRNA is detected; and it is expressed in a tissue-non-specific manner in a malignant tumor.

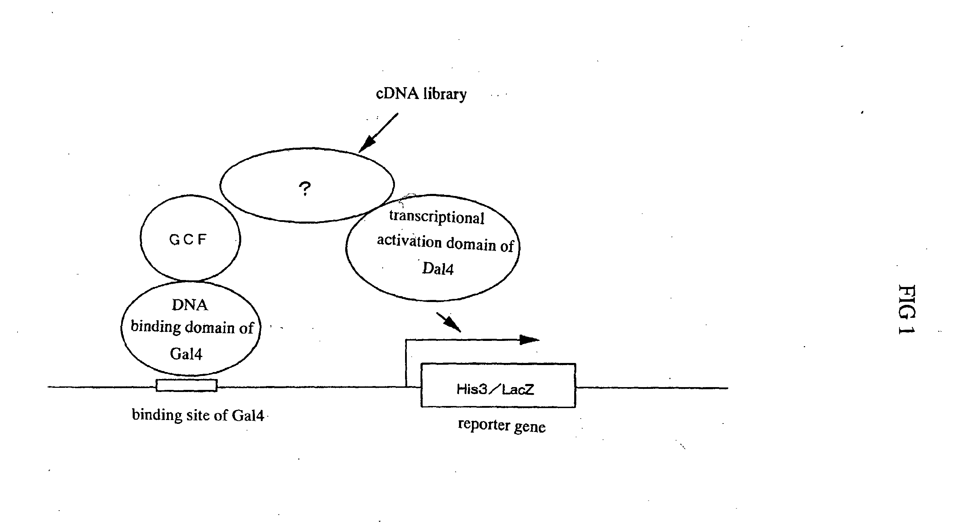

Fig. 1 schematizes a yeast Two-hybrid System using the transcription factor GCF. A transcription factor GCF is expressed as a fusion protein with a DNA-binding domain of Gal4 (GalDBD-GCF), whereas a protein derived from a cDNA library is expressed as a fusion protein with a transcriptional activation domain of Gal4. The interaction (binding) of GalDBD-GCF and a cDNA library-derived protein activates transcription of a reporter gene and thereby the expression of His3 gene and LacZ is raised. cDNA encoding a protein which interacts with GCF, therefore, can be cloned by isolating yeast in which a reporter gene is expressed. Commercially available MATCHMAKER (Clontech) and HybriZAP (Stratagene) can be adopted for performing the yeast Two-hybrid System.

As for a protein which is obtained by the above-mentioned yeast Two-hybrid System and which interacts with the transcription factor GCF, the cancer/testis antigen protein D40 is exemplified. A specific example of a cancer/testis-related gene encoding this D40 is a gene having the base sequence shown by SEQ ID NO: 1 in the sequence listing. There are two ORFs (open reading frames) in the D40 cancer/testis-related gene. Therefore, proteins as objects of the present invention specifically include: the D40 (ORF1) protein shown by SEQ ID NO:2 or the D40 (ORF2) protein shown by SEQ ID NO:3; a protein comprising an amino acid sequence in which one or a few amino acids are deleted, substituted or added in the amino acid sequence shown by SEQ ID NO:2 or 3 and which has the immunity-inducing activity; and the recombinant proteins of these. The immunity-inducing activity here means an activity inducing immune response such as antibody production, cellular immunity, immune tolerance, etc. in a host. Among these immunity-inducing activities, the T cell-inducing activity, by which the frequency of cytotoxic T lymphocyte (CTL) precursor cells is increased, is particularly preferable for a protein to possess.

There is no particular limitation to a peptide as an object of the present invention as long as it is a peptide which comprises a part of the protein of the present invention, for instance, a peptide comprising an amino acid sequence of five or more consecutive amino acids, and which binds to a histocompatibility antigen molecule (HLA) Class I. The specific examples are: a peptide comprising an amino acid sequence shown by any of SEQ ID NOs:4-111 that are derived from the aforementioned D40 (ORF1) protein or the like, e.g. Ser-Tyr-Thr-Ile-Glu-Ile-Asn-His-Arg-Leu (SEQ ID NO:36); and a peptide comprising an amino acid sequence shown by any of SEQ ID NOs:112-221 that are derived from the aforementioned D40 (ORF2) protein or the like. As for HLA Class I mentioned above, the specific examples include: HLA-A3, HLA-68, HLA-A1, HLA-A24, HLA-A0201, HLA-A0205, HLA-B7, HLA-B8, HLA-B14, HLA-B40. B1501 (B62), etc. Proteins and peptides as objects of the present invention together with the recombinant proteins and peptides to which antibodies specifically bind while such antibodies specifically bind to the said proteins and peptides, may collectively be referred to as "the present proteins/peptides". The present proteins/peptides can be prepared in accordance with the known methods based on their DNA sequence information or the like and these antigens will not be limited to those derived from human.

Genes or DNAs as objects of the present invention are specifically exemplified by: a gene encoding the D40 (ORF1) protein which comprises the amino acid sequence shown by SEQ ID NO:2; DNA encoding a protein which comprises an amino acid sequence wherein one or a few amino acids are deleted, substituted or added in the amino acid sequence shown by SEQ ID NO:2 and which has the immunity-inducing activity; a gene encoding the D40 (ORF2) protein comprising the amino acid sequence shown by SEQ ID NO:3; DNA encoding a protein which comprises an amino acid sequence wherein one or a few amino acids are deleted, substituted or added in the amino acid sequence shown by SEQ ID NO:3 and which has the immunity-inducing activity; and DNA comprising the base sequence shown by SEQ ID NO:1 or its complementary sequence and part or whole of these sequences. These genes or DNAs can be prepared by the known methods from, for instance, a human gene library or a human cDNA library based on their DNA sequence information or the like. Besides, there is no limitation to the preparation methods for cDNA which encodes the D40 protein and it can be obtained, for example, from a mRNA-derived cDNA library which is derived from HL60 or the T cell leukemia cell line Jurkat.

Further, DNA encoding a protein of the interest, which has the same effect as the D40 gene, having the immunity-inducing activity can be obtained by the hybridization with a DNA library derived from various cancer cells under a stringent condition by using the base sequence shown by SEQ ID NO:1 or its complementary sequence and part or whole of these sequences as a probe, and then by the isolation of DNA which hybridizes with the probe. A hybridization condition for obtaining the DNA is exemplified by hybridization at 42. degree. C. and washing at 42.degree. C. in a buffer solution containing 1×SSC, 0.1% SDS, and more preferable exemplification is hybridization at 65.degree. C. and washing at 65.degree. C. in a buffer solution containing 0.1×SSC 0.1% SDS. There are number of factors other than the temperature condition mentioned above that affect the hybridization stringency and those skilled in the art can actualize the same stringency as that of the hybridization referred to in the above by appropriately combining various factors.

Any fusion protein and fusion peptide may be used as a fusion protein and a fusion peptide for the present invention as long as the present proteins/peptides are bound to marker proteins and/or peptide tags. As for a marker protein, there is no limitation as long as it is a conventionally known marker protein and the specific examples include alkaline phosphatase, the Fc region of an antibody, HRP, GFP, etc. Conventionally known peptide tags including Myc tag, His tag, FLAG tag, GST tag, etc. are the specific examples of the peptide tags for the use in the present invention. These fusion proteins can be generated according to the ordinary protocols and are useful for the following: purification of the D40 protein or the like using affinity of Ni-NTA and a His tag; detection of a protein having the T cell-inducing activity; quantification of an antibody against the D40 protein or the like; use as a diagnostic marker for uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma, colon cancer, etc.; and use as a laboratory reagent in this field of art.

Antibodies that specifically bind to the aforementioned proteins and peptides of the present invention can be particularly exemplified by immune-specific antibodies such as monoclonal antibodies, polyclonal antibodies, chimeric antibodies, single-stranded antibodies, humanized antibodies, etc. These antibodies can be generated according to the ordinary protocols using a protein, such as the above-mentioned D40 etc., or part of the protein as an antigen. However, monoclonal antibodies are more preferable than the other sorts of antibodies mentioned because of their specificity. Antibodies such as the monoclonal antibodies or the like are useful not only for diagnosis and therapy, such as missile therapy, for uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma, colon cancer, etc., for instance, but for elucidating the development mechanism of malignant tumors such as oral cancer and the like.

Antibodies of the present invention described above can be created by administering to an animal (preferably non-human) the present proteins/peptides, their fragments containing epitopes, or the cells expressing the said proteins on the membrane surface, according to the conventional protocols. The monoclonal antibodies can be prepared, for instance, by any optional method such as a hybridoma method that brings antibodies produced by cultured materials of continuous cell line (Nature 256, 495-497, 1975), a trioma method, a human B-cell hybridoma method (Immunology Today 4, 72, 1983), an EBV-hybridoma method (MONOCLONAL ANTIBODIES AND CANCER THERAPY, pp.77-96, Alan R. Liss, Inc., 1985), etc.

The preparation method for a single chain antibody (US PAT.No. 4,946,778) can be adopted to prepare single-stranded antibodies to the present proteins/peptides of the present invention mentioned above. Besides, transgenic mice, other mammals, etc. can be used for expressing humanized antibodies, clones expressing the present proteins/peptides can be isolated/identified using the antibodies mentioned above, and their polypeptides can be purified by affinity chromatography. Antibodies to the present proteins/peptides or to their peptides containing antigenic epitopes can possibly be used for diagnosis and therapy for various cancers as described earlier. Furthermore, recombinant proteins or peptides to which these antibodies specifically bind are also covered by the present proteins/peptides of the present invention as described earlier.

The functions of the present proteins/peptides can be analyzed by using, for example, antibodies such as the aforementioned monoclonal antibodies and the like that are labeled with fluorescent materials such as FITC (Fluorescein isothiocyanate), tetramethylrhodamine isothiocyanate, etc., radioisotopes such as ¹²⁵I, ³²p, ¹⁴C, ³⁵S, ³H, etc., or enzymes such as alkaline phosphatase, peroxidase, â-galactosidase, phycoerythrin, etc. and by using fusion proteins fused with fluorescent proteins such as Green Fluorescent Protein (GFP), etc. As for immunological detection methods using the antibodies of the present invention, RIA method, ELISA method, fluorescent-antibody method, plaque method, spot method, haemagglutination, Ouchterlony method, etc. are exemplified.

The present invention also relates to a host cell comprising an expression system capable of expressing the present proteins/peptides of the above. Genes encoding the present proteins/peptides can be introduced into a host cell by the methods described in many standard laboratory manuals such as manuals of Davis et al. (BASIC METHODS IN MOLECULAR BIOLOGY, 1986) and of Sambrook et al. (MOLECULAR CLONING: A LABORATORY MANUAL, 2nd Ed., Cold Spring Harbor Laboratory Press, Cold Spring Harbor, N.Y., 1989), and the examples include calcium-phosphate transfection, DEAE-dextran-mediated transfection, transvection, microinjection, cationic lipid-mediated transfection, electroporation, transduction, scrape loading, ballistic introduction, infection, etc. The examples of host cells include bacterial prokaryotic cells such as E. coli, Streptomyces, Bacillus Subtilis, Streptococcus, Staphylococcus, etc., eukaryotic cells such as yeast, aspergillus, etc., insect cells such as Drosophila S2, Spodoptera Sf9, etc., animal cells such as L cell, CHO cell, COS cell, HeLa cell, C127 cell, BALB/c3T3 cell (including mutants deficient in dihydrofolate reductase, tymidine kinase, etc.), BHK21 cell, HEK293 cell, Bowes malignant melanoma cell, etc. and plant cells or the like.

There is no limitation to an expression system as long as the expression system is capable of expressing the present proteins/peptides described above in a host cell and the examples include chromosome-, episome- and virus-derived expression systems, for instance, vectors derived from bacterial plasmid, vectors derived from yeast plasmid, vectors derived from papovavirus such as SV40, vaccinia virus, adenovirus, varicella virus, pseudorabies virus, retrovirus, and vectors derived from bacteriophage or transposon and vectors derived from the combination of these two, e.g. vectors derived from genetic factors of plasmid and bacteriophage, such as cosmid and phagemid. The expression systems may comprise a control sequence for regulating the expression in addition to a sequence for raising the expression.

Host cells comprising the above-mentioned expression systems and the cell membranes of the cells, or the present proteins/peptides obtained by culturing the cells can be used in the screening methods of the present invention as described below. For example, the method of F. Pietri-Rouxel et al. (Eur. J. Biochem., 247, 1174-1179, 1997) or the like can be used to obtain cell membranes. Further, to collect and purify the present proteins/peptides from the cell culture, the known methods can be adopted including ammonium sulfate- or ethanol-precipitation, acid extraction, anion- or cation-exchange chromatography, phosphocellulose chromatography, hydrophobic interaction chromatography, affinity chromatography, hydroxyapatite chromatography and lectin chromatography, where the high performance liquid chromatography is preferably used. As a column especially used for affinity chromatography, columns to which antibodies to the present proteins/peptides are bound, for instance, are used, and when ordinary peptide tags are added to the present proteins/peptides mentioned above, columns to which substances having affinity with the peptide tags are bound are used in order to obtain the present proteins/peptides. The purification methods for the present proteins/peptides mentioned above may also be employed for peptide synthesis.

In the present invention, a non-human animal whose gene function to encode the present proteins/peptides mentioned above is deficient on its chromosome means a non-human animal part or whole of whose gene on its chromosome encoding the present proteins/peptides is inactivated by gene mutations such as destruction, deletion, substitution, etc. so that whose function to express the present proteins/peptides is lost. Further, a non-human animal which over-expresses the present proteins/peptides is specifically exemplified by a non-human animal which produces larger amount of the present proteins/peptides than a wild-type non-human animal does. Although rodents or the like such as mice, rats, etc. are particularly exemplified for non-human animals of the present invention, the examples will not be limited to these animals only.

Homozygous non-human animals that are born according to Mendel's Law include the deficient type or the over-expressing type for the present proteins/peptides as well as their wild type littermates. By using the deficient type animals or the over-expressing type animals of these homozygous non-human animals together with their wild-type littermates at the same time, accurate comparative experiments can be carried out on the individual level. In performing screening of the present invention described below, it is, therefore, preferable to use the wild type non-human animals, i.e. animals of the same species as, or even better the littermates of, non-human animals whose gene function to encode the present proteins/peptides is deficient or over-expressing on their chromosomes in parallel with the deficient or over-expressing type animals. The method of producing a non-human animal whose gene function to encode the present proteins/peptides is deficient or over-expressing on its chromosome is now explained in the following with a D40 knockout mouse and a D40 transgenic mouse as examples.

A mouse, for instance, whose gene function to encode the D40 protein is deficient on its chromosome, i.e. a D40 knockout mouse is generated by the following steps. A gene encoding mouse D40, which is homologous to human D40, is screened by using a gene fragment obtained by a method such as PCR or the like from the mouse gene library. A screened gene which encodes mouse D40 is subcloned with a viral vector or the like and is identified by DNA sequencing. Then whole or part of a gene of this clone which encodes mouse D40 is substituted with a pMC1 neo gene cassette or the like. A gene such as a diphtheria toxin A fragment (DT-A) gene, a herpes simplex virus tymidine kinase (HSV-tk) gene, etc. is introduced onto the 3'-end, and thus a targeting vector is constructed.

The targeting vectors thus constructed are linearlized and introduced into ES cells by electroporation or the like to cause homologous recombination. Among the homologous recombinants, ES cells in which homologous recombination have occurred are selected by the use of antibiotics such as G418, ganciclovir (GANC), etc. It is preferable to confirm whether the ES cells selected are the recombinants of the interest by Southern blotting or the like. A clone of the ES cells confirmed is microinjected into a mouse blastocyst and which blastocyst is placed back to the recipient mouse to generate a chimeric mouse. A heterozygous mouse can be obtained by intercrossing the chimeric mouse and a wild type mouse. By further intercrossing the heterozygous mice, the D40 knockout mice of the present invention can be generated. Whether the ability of expressing D40 is lost in a D40 knockout mouse is examined by Northern blotting upon isolating RNA from the mouse obtained by the above-described method and by Western blotting or the like with which the D40 expression in the mouse can be directly examined.

A D40 transgenic mouse is created by the following steps. A promoter such as chicken â-actin, mouse neurofilament, SV40, etc. and poly (A) such as rabbit â-globin, SV40, etc. or introns are fused with cDNA encoding D40 derived from human, mouse, rat, rabbit, etc., to construct a transgene. This transgene is microinjected into the pronucleus of a mouse fertilized egg. After the obtained egg cell is cultured, it is transplanted to the oviduct of the recipient mouse which was fed thereafter. Neonetal mice that have the aforementioned cDNA were selected from among all the mice born and thus the transgenic mice are created. Neonatal mice having the cDNA can be selected by extracting crude DNA from the mice tails or the like and then by a dot hybridization method using a gene encoding the introduced D40 as a probe and by PCR method or the like using a specific primer.

Genes or DNA encoding the present proteins/peptides mentioned above, the present proteins/peptides, fusion proteins in which the present proteins/peptides and marker proteins and/or peptide tags are bound, antibodies to the present proteins/peptides, host cells comprising expression systems capable of expressing the present proteins/peptides, etc. are useful, as specifically explained below, for therapy and diagnosis for uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma, colon cancer, etc. These can be used for not only screening a promoter or a suppressor for the immunity-inducing activity or a promoter or a suppressor for expression of the present proteins/peptides. but elucidating the mechanism of immune response such as induction of cytotoxic T cells (including all the T cells such as CD4 antigen-positive T cells, CD8 antigen-positive T cells, helper T cells, killer T cells, suppressor T cells, etc.), or the like.

As for a screening method for a promoter or a suppressor for the immunity-inducing activity of the present invention, methods are exemplified that use the following: the foregoing present proteins/peptides of the present invention or a cell membrane which expresses the present proteins/peptides, and a test substance; a cell which expresses the present proteins/peptides and a test substance; a non-human animal such as a knockout mouse or a transgenic mouse for the present proteins/peptides and a test substance; and etc. Further, a method using a cell which expresses the present proteins/peptides mentioned above and a test substance, a method using a non-human animal such as a knockout mouse and a transgenic mouse for the present proteins/peptides and a test substance, etc. can be employed for a method of screening a promoter or a suppressor for expression of the present proteins/peptides.

A screening method wherein the above-mentioned present proteins/peptides or a cell membrane that expresses the present proteins/peptides are used along with a test substance is specifically exemplified by a method wherein the present proteins/peptides or the present proteins/peptides expressed on the surface of a cell membrane is made to contact a test substance and wherein the immunity-inducing activity of the present proteins/peptides are measured and assessed. Further, as for a screening method wherein the cells expressing the present proteins/peptides and a test substance is used, a method is specifically exemplified in which a cell expressing the present proteins/peptides is made to contact a test substance and in which the immunity-inducing activity of the present proteins/peptides and the variation in the expression amount of the present proteins/peptides are measured and assessed.

As for a screening method wherein a non-human animal whose gene function to encode the aforementioned present proteins/peptides is deficient on its chromosome, or a non-human animal which over-expresses the present proteins/peptides is used along with a test substance, the following methods are specifically exemplified: a method wherein a cell or a tissue from these non-human animals is made to contact a test substance in vitro to measure and assess the immunity-inducing activity of the present proteins/peptides and the variation in the expression amount of the present proteins/peptides; a method wherein a non-human animal whose gene function to encode the aforementioned present proteins/peptides is deficient on its chromosome or a non-human animal which over-expresses the present proteins/peptides is administered with a test substance in advance and wherein the immunity-inducing activity of the present proteins/peptides and the variation in the expression amount of the present proteins/peptides in a cell or a tissue obtained from the non-human animal are measured and assessed; or a method wherein a non-human animal whose gene function to encode the aforementioned present proteins/peptides is deficient on its chromosome or a non-human animal which over-expresses the present proteins/peptides is administered with a test substance in advance and wherein the immunity-inducing activity of the present proteins/peptides and the variation in the expression amount of the present proteins/peptides in the non-human animal are measured and assessed.

The screening methods using a test substance mentioned above and the present proteins/peptides will be explained below with the specific examples, however, the screening methods of the present invention will not be limited to these examples. As for a method for measuring and assessing the immunity-inducing activity of the present proteins/peptides by contacting the above-mentioned test substance with the present proteins/peptides, a method is specifically exemplified wherein the test substance and the present proteins/peptides are made to contact in the presence of tumor-infiltrating T cells which are largely proliferated in vitro from peripheral lymphocytes by using ordinary interleukin-2, and wherein the increase and decrease of the inducing activity of T cells such as CTL is measured and then the results are compared for the assessment with that of the control in which the test substance is absent. Further, as for a method of measuring and assessing the variation in the expression of the present proteins/peptides by contacting a test substance with a cell membrane or a cell which expresses the present proteins/peptides as mentioned above, a method is specifically exemplified wherein a cell expressing the present proteins/peptides is cultured in the presence of a test substance and wherein the increase or decrease of the present proteins/peptides that are expressed on the surface of the cell membrane after culturing for a certain period of time is assessed either by the immunochemical detection by ELISA or the like with the use of antibodies that specifically bind to the present proteins/peptides of the present invention, or by using the suppression or promotion of the mRNA expression as an index. Detection of mRNA, mentioned above, can be carried out by such as DNA chip method, Northern hybridization method, etc. Alternatively, when a cell introduced with a gene to which a reporter gene such as luciferase, etc. is linked in the downstream of the promoter of a gene encoding the present proteins/peptides is used, suppression or promotion, caused by a test substance, of the expression of the gene encoding the present proteins/peptides can be detected with the activity of the reporter gene as an index.

A promoter for the immunity-inducing activity or an expression promoter of the present invention obtained by the aforementioned screening methods can be applied to the therapy and the like for patients in need of the promotion of the immunity-inducing activity or promotion of expression of the present proteins/peptides. Meanwhile, a suppressor for the immunity-inducing activity or an expression suppressor of the present invention obtained by the aforementioned screening methods can be applied to the therapy and the like for patients in need of the suppression of the immunity-inducing activity or the expression of the present proteins/peptides. The present proteins/peptides of the present invention and the antibodies against these can be employed for an active ingredient in anti-tumor agents for uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma, colon cancer, etc. For example, when the present proteins/peptides are injected orally, intravenously, intradermally, subcutaneously, etc., an anti-tumor effect can be expected due to the increase in the in vivo T cell-inducing activity. Furthermore, the above-mentioned antibodies can be used in a missile therapy.

There is no limitation to a detection method of the present invention for cytotoxic T cells (activated T cells) or their precursor cells as long as the method uses a HLA molecule and a present peptide and the examples include a method wherein a complex of a HLA molecule, such as a HLA Class I molecule or the like, and a present peptide is used, and a method wherein a complex of a HLA molecule, such as a HLA Class I molecule or the like, and a present peptide is formed on the cell surface or on the surface of a carrier such as a fluorescent microparticle or the like. A method is exemplified, for instance, according to the method described in the literature (Science, 274, 94-96, 1996), wherein a complex of a HLA Class I molecule and a present peptide is formed on the surface of a fluorescent microparticle, a T lymphocyte cell from peripheral blood of a cancer patient is made to contact and bind to the complex, and then T cells bound to HLA-peptide complexes that are formed on the surface of the microparticles are detected. Further, there is no limitation to a detection reagent of the present invention for cytotoxic T cells (activated T cells) or their precursor cells as long as a reagent contains HLA molecules and the present peptides where the examples are a reagent containing a complex of a HLA molecule such as a HLA Class I molecule or the like and a present peptide and a reagent containing a complex of a HLA molecule such as a HLA Class I molecule or the like and a present peptide along with a carrier such as a fluorescent microparticle or the like.

Further, activated T cells can be induced upon the in vitro stimuli with the present proteins/peptides of the present invention. For instance, the tumor-responsive activated T cells are induced when peripheral lylmphcytes or tumor-infiltrating lymphocytes are stimulated with the present proteins/peptides and IL-2. These activated T cells can be used effectively in an adoptive immunotherapy. Besides, by expressing the present proteins/peptides either in vivo or in vitro in dendritic cells that are strong antigen-presenting cells, immunity can be induced by injecting these antigen-expressing dendritic cells.

Further, whole or part of the antisense chain of DNA or RNA encoding the present proteins/peptides of the present invention can be made a diagnostic probe for cancer. Furthermore, cancers such as uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma, colon cancer, etc. can be diagnosed by the use of this diagnostic probe for cancer and a diagnostic drug of the present invention containing an antibody which specifically binds to the present proteins/peptides. As for the above-mentioned diagnostic probe, a probe which comprises whole or part of the antisense chain of DNA (cDNA) or RNA (cRNA) encoding the present proteins/peptides and which has enough length to be established as a probe (at least equal to or more than 20 bases) is preferable. The specific examples of test samples used for detection are genomic DNA, RNA or cDNA from the cells of subjects that are obtained from, for example, biopsy of such as blood, urine, saliva, tissues, etc. However, the test samples will not be limited to these examples and amplified test samples by the means of PCR or the like may also be used.

The present invention will be specifically explained in the following with reference to the examples, but the technical scope of the invention will not be limited to these examples.

### Example A. [Materials and methods]

### A-1 (Culture cell lines and tumor samples)

Culture cell lines used are shown in Table 3. These cell lines are maintained in RPMI 1640 containing 10% bovine fetal serum and 0.3% glutamine or in DMEM (Dulbecco Modified Eagle Medium) and are cultured at 37.degree. C. under 5% CO₂. The primary tumor samples are obtained from operations performed at Department of Oral Surgery, School of Dental Medicine, Hokkaido University and Department of Obstetrics and Gynecology, Second Department of surgery, and First Department of Internal medicine of School of Medicine, Hokkaido University. They were immediately frozen in liquid nitrogen and kept at □80. degree. C. until used.

**Table 3**

| Cell type | Culture cell line | Expression |
|---|---|---|
| Normal fetal pulmonary fibroblast | HFL | - |
| Uterine cervix cancer | HeLa | + |
| Epithelial-like cancer | A431 | + |
| T cell tumor | Jurkat | + |
| Promyelocytic leukemia | HL60 | + |
| Esophageal cancer | TE-8 | + |
| Pancreatic cancer | PC143c | + |
| Malignant melanoma | SkMEL | + |
| | AKI | + |
| | G361 | + |
| Lung cancer | PI10 | + |
| | NCIH226 | + |
| | RERF-LC-MS | + |
| | RERF-LC-OK | + |
| Oral cancer | HSC-2 | + |
| Breast cancer | MCF-7 | + |
| Bladder cancer | DAB-1 | + |
| | T-24 | + |
| | UMUC-2 | + |

### A-2 (cDNA cloning of D40)

Two thirds of carboxyl terminal of the coding region of transcription factor GCF was incorporated into pGBT-9 (Clontech) and the resulting plasmid pGAL-GCF was used as a bait for screening by the yeast Two-hybrid System (see Fig. 1). First, pGAL-GCF was transformed to the yeast Y153 line (Genes Dev.7, 555-69, 1993), with which a cDNA library of an immortalized human B cell line was screened. Taking a clone which was positive to histidine- and LacZ-phenotype, its binding specificity with GCF was examined and its base sequence was identified. In order to obtain a longer clone by using this clone, a cDNA library derived from the human promyelocytic leukemia cell line HL60 was screened. Further, in accordance with the protocol attached to the 5'/3' RACE kit (Beohringer/Roche), 5' RACE (Rapid Amplification of cDNA ends) reaction was carried out using the directed primer (Proc. Natl. Acad. Sci. USA 85, 8998-9002, 1988), that is, total RNA derived from HL60 and the T cell leukemia cell line Jurkat were used along with the D40-specific reverse primer MT 194 (5'-GAGTCCTCGGTGTGAAGCTTTA-3': SEQ ID NO:222) for cDNA synthesis of the first strand. Deoxyadenosine was added to 3' end of the cDNA and an oligo dT anchor primer and a D40-specific reverse primer MT195 (5'-ACAGTGTGACTTGATGTCAGGT-3'; SEQ ID NO:223) were used for PCR.

### A-3 (DNA sequencing and homology search)

Plasmid DNA was purified in a Qiagen column (Qiagen) and used as a template for the cycle sequencing reaction in performing Dye terminator method (Proc. Natl. Acad. Sci. USA 74, 5463-5, 1977), then the base sequence was determined on a fluorescent DNA sequencer (373 genetic analyzer, ABI). Sequence homology was studied with the disclosed data bases.

### A-4 (Chromosome mapping)

Fluorescence in situ hybridization (FISH) was performed in accordance with a previously reported procedure (Sambrook J, Fritsch EF, Maniatis T (1989): Molecular Cloning, Cold Spring Harber Press, New York, p7.39). Metaphase spreads were prepared by incorporating bromodeoxyuridine (BrdU) into the concanavalin-A stimulated lymphocytes of a human male which were transformed with EB virus. A mixture of plural cDNA clones over the full length of D40cDNA (200 ng) was labeled with biotin. This cDNA as a probe was reacted with samples of R-banded metaphase chromosomes for 48 hours at 37.degree. C. Subsequently, the chromosome samples were sequentially washed in 50% formamide, first at 0.5×SSC and then at 2×SSC. Then, FISH signals were amplified with an anti-biotin antibody (3 ìg/ml, Vector) and a FITC anti-goat antibody (40 ìg/ml, American Qualex). Further, FISH signals were amplified with the anti-FITC-Alexa488 (40 ìg/ml, Molecular Probes).

### A-5 (In vitro transcription and translation)

A reaction was occurred in the presence of 35S-methionine using a plasmid pBS-D40 having all coding regions of D40cDNA in pBluescript KS( ), a T7 RNA polymerase and the TNT rabbit reticulocyte lysate (Promega). The translation products were analyzed on SDS-PAGE and detected by the image analyzer BAS2000 (Fuji Film).

### A-6 (Western blotting)

Cell lysate was prepared using TNE buffer (10 mM Tris-Cl, pH 7.8, 1% NP40, 150 mM NaCl, 1 mM EDTA, 0.5 mM PMSF, 10 mg/ml Aprotinin, 10 mg/ml Leupeptin), which was then analyzed on a 8% SDS-PAGE and fixed on a nitrocellulose filter. An anti-Flag monoclonal antibody (M2: Kodak) and a peroxydase-conjugated goat anti-mouse immunoglobulin (Jackson ImmunoRes.) were used as the first and the second antibodies, respectively. Signals were detected by ECL system (Amersham).

### A-7 (RNA separation)

Total RNA was separated and purified from the cell lines and tumor samples by the method of acid guanidinium thiocyanate-phenol-chloroform extraction (Anal. Biochem. 162, 156-159, 1987). RNA was purified from approximately 1×10⁷ cells and 80 mg of a tumor tissue using 2 ml of Trizol reagent (Life Technology).

### A-8 (Northern blotting)

Northern blot analysis was performed in accordance with a previously described method (Genomics 32, 483-484, 1996). The hybridization took place overnight at 37.degree. C. in a solution containing 50% formamide, 5×Denhardt solution and 0.5% SDS. The filter membrane was sequentially washed under the gradually increasing stringency. The final washing was done under 0.2×SSC and 0.1% SDS at 48. degree. C., and then the signals were detected by the BAS2000.

### A-9 (RT-PCR)

1 ig of the total RNA was reversely transcribed to cDNA using Superscript II RNase H-reverse transcriptase (Life Technology). The PCR reaction was occurred in a pH8.3 buffer containing 10 mM Tris-HCl, 1.5 mM MgCl₂ and 50 mM KCl using: 0.1 ìg of cDNA; 10 pmol each of specific oligonucleotide primers (forward: MT1149; 5'-CACATCCAGTGAGACCA-3'; SEQ ID NO:224, reverse: MT152; 5'-TCCCATCTTCTGATGTG-3'; SEQ ID NO:225, or forward: MT227; 5'-CAGGAATCCAATGCTTGG-3'; SEQ ID NO;226, reverse: MT188; 5'-CTGTTGCAGGTAAGATC-3'; SEQ ID NO:227); and 0.5 unit of Taq polymerase. The RT-PCR was carried out using a â-actin primer in order to check the integrity of RNA.

### A-10 (Cytotoxity test)

Peripheral blood of a patient whose D40 gene expression in the tumor tissue was observed by RT-PCR method was multi-layered in Ficoll-Conray solution with specific gravity of 1.082 and the blood was separated to plasma, lymphocyte phase, Ficoll-Conray phase and erythrocyte phase from the top by centrifugation of 1500 rpm for 30 min. The lymphocyte phase was collected and cultured for a day in AIM-V, a serum-free medium, after which the cell population adhered to the bottom of the culture flask and the suspended cell population were separated by gently shaking the flask. The adhered cell population is a cell population called PBMC (peripheral blood mononuclear cells) which includes B cells, macrophages and dendritic cells that are so-called antigen-presenting cells (APC). The APC were cultured for four days in AIM-V+GM-CSF, a culture condition for dendritic cells which have particularly high antigen-presenting ability among the APC mentioned above. Thereafter, a test peptide was added to this APC culture solution at the concentration of 1 ìM, which was then cultured for two days. When this culture period was over, the culture solution was replaced with AIM-V+TNF-α+INF-α, and the APC were cultured for another two days. The APC after the culture were treated with radioactivity in order to get rid of their proliferation ability.

On the other hand, the suspended cell population mainly contains T cells and these suspended cells were cultured in AIM-V+IL-2 for four days. Then CD8-positive T cells were selected using magnetic beads in which anti-CD8 antibodies were solid-layered and the CD8-positive T cells obtained were cultured for four days. These CD8-positive T cells were added to APC, which have been treated with radioactivity as mentioned above, and cultured for two days. This operation, named MLPC (Mix lymphocyte peptide culture) by the present inventors, was repeated for two or three times and the CD8-positive T cells obtained were used for the cytotoxity test. The CD8-positive T cells which had undergone MLPC twice as described above were used as effecter cells and C1R-A24 cells incorporated with ⁵¹Cr were used as target cells in the cytotoxity test. 5×10² target C1R-A24 cells added with 1 ìM of a test peptide were co-cultured with 2.5×10³ effecter T cells for six hours. Then the radioactivity of ⁵¹Cr released from the target C1R-A24 cells were measured and the cytotoxity activity (%) was calculated. The case when a test peptide was not added was made the control.

### Experiment B [Results]

### B-1 (Identification and cloning of D40)

Proteins that specifically bind to the transcription factor GCF were screened by the yeast Two-hybrid System. As described in Example A-2 above, a plasmid pGAL-GCF which expresses a fusion protein of a DNA-binding domain of Gal4 and GCF was introduced into the yeast Y153 line, which was then used to screen the human B cell-derived cDNA library. Nine among 12×10⁵ yeast clones that underwent search were positive to histidine- and LacZ-phenotypes. One among the nine clones was named D40 and its binding specificity with GCF was examined. The result showed that a protein encoded by this clone did not bind to proteins such as lamin, p53, etc., thus revealing that the binding of D40 and GCF was a specific one.

Upon determination of the DNA base sequence of this clone, ORF was found which, however, was devoid of a termination codon on 5' side of the codon corresponding to methionine on the amino end, although it had a termination codon on 3' side. In order to obtain longer clones, the cDNA library of the human promyelocytic leukemia cell line HL60 was screened with the D40cDNA clone as a probe. Some clones were isolated as a result, however, none of them were shown to have a termination codon on 5' side. Hence, the 5'RACE (Rapid amplification of cDNA ends) method was carried out using total RNA derived from HL60 and the T cell leukemia cell line Jurkat. With this method, a clone having a termination codon for a reading frame of the 5' end was successfully obtained (ORF1) . In addition to this, a clone containing the 3' end region was obtained and its base sequence was determined, which proved the existence of a sequence encoding another protein (ORF2) on the 3' side of ORF1. The total cDNA sequence of D40 is shown as SEQ ID NO: 1, the amino acid sequence of the D40 (ORF1) protein is shown as SEQ ID NO:2 and the amino acid sequence of the D40 (ORF2) protein is shown as SEQ ID NO:3, respectively.

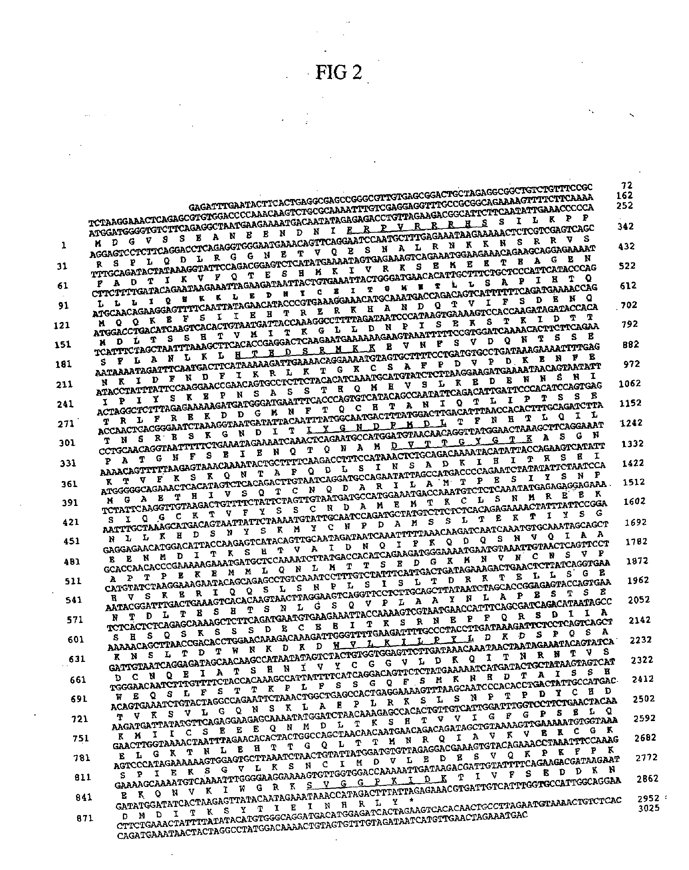

Part of the base sequence of D40 (ORF1) cDNA and the ORF1 amino acid sequence coded by that base sequence part are shown in Fig. 2. In Fig. 2, the amino acid sequence is shown by single letters under the base sequence, the mark * indicates the termination codon and the numbers on left and right hand sides of the sequences indicate positions in the amino acid sequence and the base sequence, respectively. Besides, the leucine zipper-like sequence (Science 240, 1759-64, 1988) are shown in bold letters and the examples of peptides having high affinity with various human histocompatibility antigens (HLA) Class I molecules (see Table 4) are underlined, respectively. There are some sequences that very closely resemble a peptide motif which binds to a HLA Class I molecule (Immunogenet. 41, 178-228, 1995), in the amino acid sequence of the D40 (ORF1) protein as shown in Fig. 2 or the D40 (ORF2) protein mentioned above. According to the program which predicts amino acid sequences binding to HLA Class I molecules and which scores such prediction (J. Immunol. 152:163. 1994. Web site; HLA Peptide Binding Predictions; http://bimas.dcrt.nih.gov/molbio/hla bind/). scores of the underlined amino acid sequences in D40 (ORF1) in Fig. 2 were calculated. The results are shown in Table 4. Some peptides having high affinity with HLA Class I molecules as mentioned above are shown in Table 4. The scores for the amino acid sequences of these peptides are shown to be equal to or higher than that of the MAGE3 sequence which have already been shown to bind to a HLA-A1 molecule and proved to be recognized by a cytotoxic T lymphocyte. Therefore, it is understood that the underlined amino acid sequences in D40 (ORF1) in Fig. 2 have antigenicity. Further, an amino acid sequence shown by any of SEQ ID NOs:4-111 in the D40 (ORF1) protein and an amino acid sequence shown by any of SEQ ID NOs:112-221 in the D40 (ORF2) protein also scored high marks in the above-mentioned scoring so that these sequences are very likely to possess antigenicity.

**Table 4**

| HLA Class I | D40-derived peptide | Score |
|---|---|---|
| A1 | HTEDSRMKK | 225 |
| A0201 | WVLKILPYL | 402 |
| A3 | LLDNPISEK | 45 |
| A24 | IYGNDFMDL | 240 |
| A0205 | WVLKILPYL | 252 |
| A68 | DVTTGYGTK | 360 |
| A68 | SVGGPKIDK | 240 |
| B7 | PPRSPLQDL | 120 |
| B8 | WNKDKDWVL | 120 |
| B14 | ERPVRRRHS | 300 |
| B40 | CEITGMNTL | 80 |

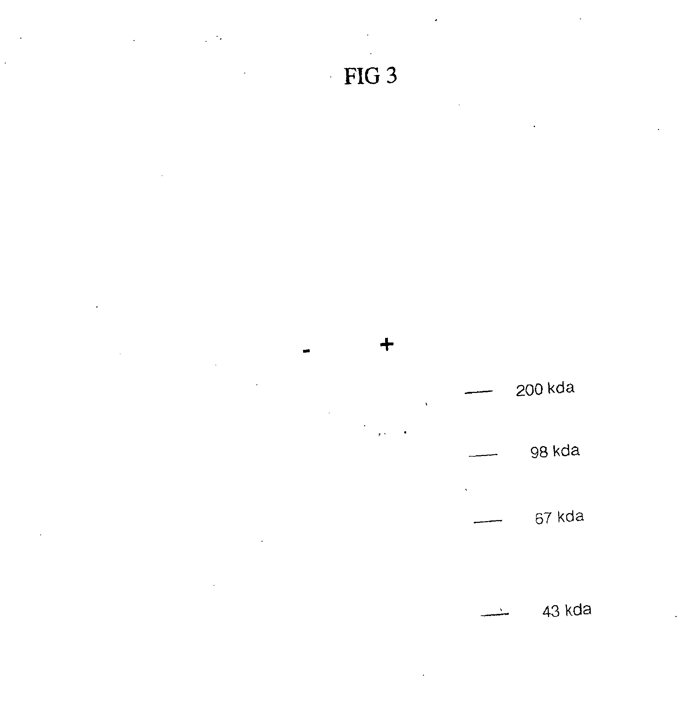

As shown in Fig. 2, ORF1 in D40cDNA encodes 887 amino acids. To confirm the accuracy of ORF1, in vitro transcription and translation reactions were occurred in accordance with the method described in the Example A-5 by using reticulocyte lysate which is well known as an in vitro synthesis system for proteins. cDNA in the whole coding region of the D40 (ORF1) protein was incorporated into a plasmid having a T7 RNA promoter. The reaction was occurred using this plasmid in a manner described in the Example A-5. The result is shown in Fig. 3. In Fig. 3, (+) and (-) indicate presence or absence of a plasmid having D40cDNA and the numbers on the right of the gel indicate molecular weight markers. The result of the gel analysis for the translated protein exhibited two bands between the molecular weights of 110-130 KD.

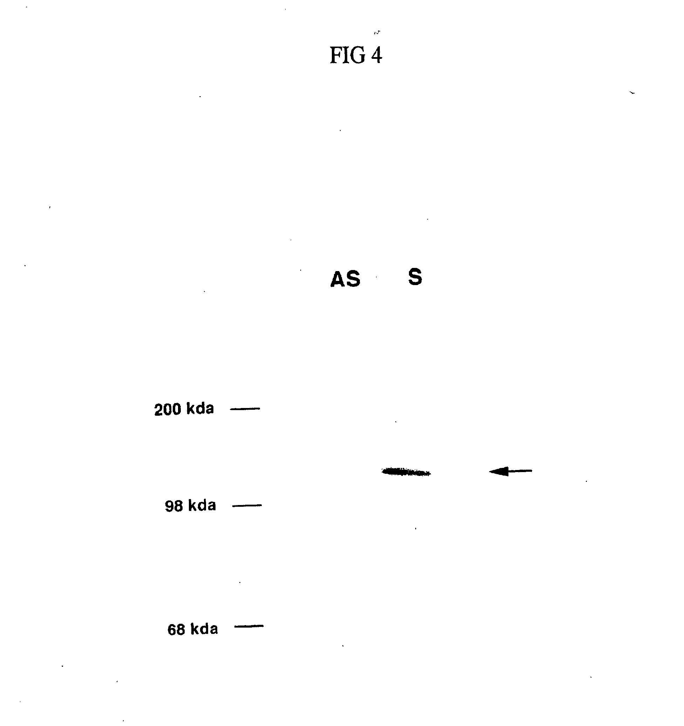

Next, in vivo expression of the protein encoded by D40 (ORF1) was examined. A plasmid, which expresses the D40 (ORF1) protein added with a Gal4 DNA-binding domain to the amino end of D40 (ORF1) protein as a tag (FLAG), was introduced into a Cos7 cell using lipofectamine. 48 hours later, the Cos7 cell extract was prepared which underwent Western blotting using an anti-flag antibody as described in the Example A-6. Fig. 4 shows the result. In Fig. 4, S and AS indicate that D40cDNA was cloned into a plasmid in a sense and antisense way respectively, and the numbers on the gel left indicate the molecular weight markers. As shown in Fig. 4, a band with the molecular weight of 120 KD was detected.

Homology was searched with each data base of GenBank/EMBL/DDBJ as mentioned in the Example A-3, and no protein having high homology with the D40 (ORF1) protein was found. Although it did not show high homology, the D40 (ORF1) protein had a faint homology with some proteins such as Synaptonemal complex protein 1 (SCP1), Paramyosin, etc. These proteins rich in α-helix are thought to be fiber proteins which bind intrecellular and extracellular structures together (EMBO J 11, 5091-5100, 1992). Therefore, there is a possibility that the D40 protein is a protein which, having associated with a fiber protein, contributes to the construction of the cell structure which is only observed in a testis cell or of the cell structure which is observed in the ectopic expression in a cancer cell. As mentioned earlier, SCP1 is similar to D40 also in that it exists in a human autosome. Further, homology search was similarly performed, but no protein having high homology with the D40 (ORF2) protein was found.

### B-2 (Localization of D40 on chromosomes)

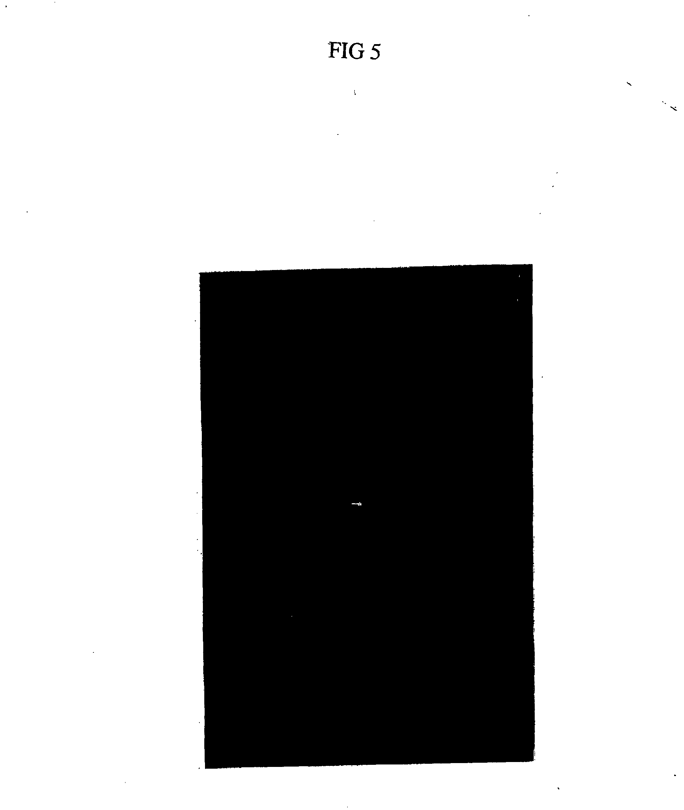

Localization of D40 genes on chromosomes was determined by fluorescent in situ hybridization. As shown in Fig. 5, signals were evident near the centromere region of human chromosome 15, i.e. in the long arm q14-15. Out of 49 cells examined, fluorescence signals in this region were observed on all four chromatids in 1 cell, on three chromatids in 3 cells, on two chromatids in 30 cells and on one chromatid only in 15 cells. No reproducible fluorescence signal was detected on other chromosomes. Localization of D40 genes on this chromosome was also confirmed in the simultaneous hybridization experiment using an a-satellite DNA probe which is specific to chromosome 15. These results suggest that D40 genes localize on human chromosome 15q14-15.

### B-3 (Expression of D40mRNA in normal human tissues)

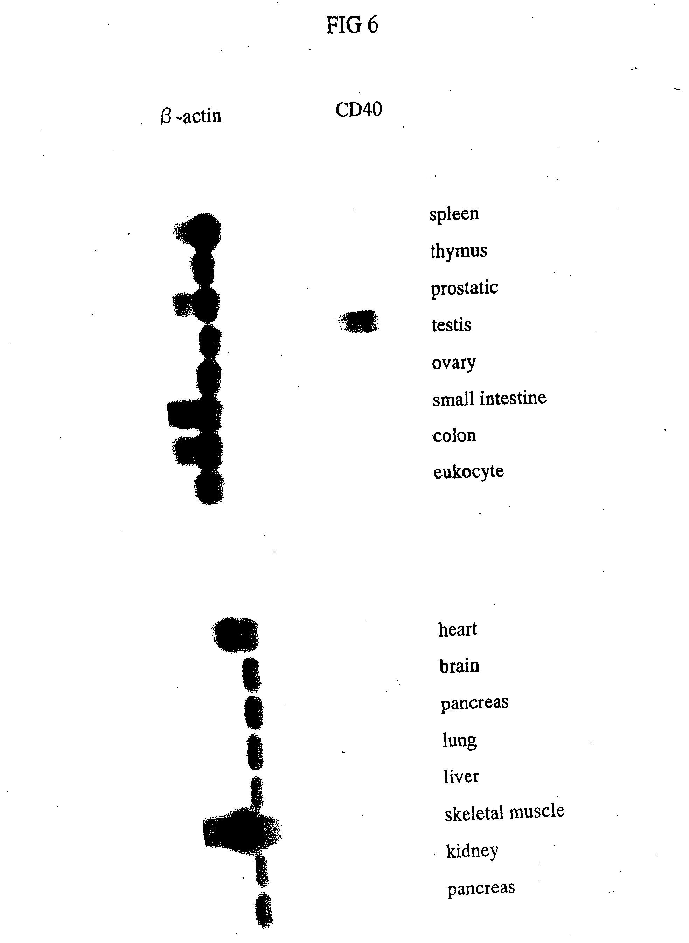

The expression of D40 genes in normal human tissues was first examined by Northern blot method. Fig. 6 shows the result of Northern blotting for a human multiple tissue mRNA blot membrane (Clontech). As is shown in Fig. 6, frequent expression of D40mRNA was observed in the testis. Besides, about 5 Kb of a major transcript and about 3.5 Kb of a minor transcript were detected in the testis. Although the expression was equal to or less than 1/50 of that observed in the testis, a small amount of D40mRNA expression was observed in the placenta other than in the testis. However, expression of D40 was not at all detected in other normal human adult tissues other than those two (Fig. 6, top). But when the probe was taken out from the same membrane which was then hybridized with a probe for â-actin, D40 was confirmed to be expressed in all the tissues (Fig. 6, bottom).

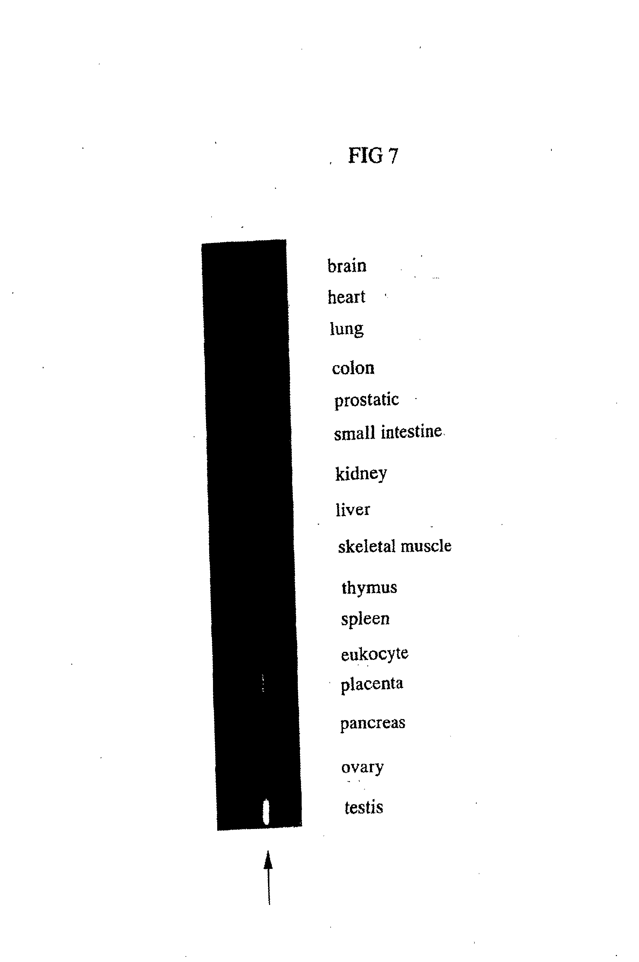

The result of the Northern blotting was next confirmed by RT-PCR method which exhibits higher sensitivity than Northern blot method does. Fig. 7 shows the result of RT-PCR method performed with a human multiple tissue cDNA (Clontech). The arrow in Fig. 7 indicates specific PCR products. Frequent expression of D40 was observed in the testis in the RT-PCR result. D40 expression was not detected in other normal human tissues except that a faint expression was observed in the placenta. These results suggest the selective expression of D40mRNA in the testis in normal human tissues.

### B-4 (D40 expression in cancer cell lines and primary cancers)

D40 expression was examined in cancer cells. First, D40 expression in various cancer cell lines was studied by RT-PCR method. The results are shown in Table 3. As is shown in Table 3, D40 was observed to be expressed in all the cancer cell lines examined including malignant melanoma, lung cancer and digestive system-derived cancers. However, the expression was not detected in the normal fetal pulmonary fibroblast line. Expression frequency of D40 in human primary cancers was then examined. Table 5 shows the results. As is clear from Table 5, expression of D40mRNA was observed in most of samples for primary cancers such as oral cancer, uterine cancer, lung cancer, etc. Oral cancer among these cancers expressed D40 in about 63% of the incidents examined. This was the highest frequency in the primary cancers examined. The expression was also observed in uterine cancer (44%), lung cancer (40%), ovarian cancer (36%), pancreatic cancer (27%), neuroblastoma (20%) and colon cancer (13%), but not in cholangiocarcinoma, gastric cancer and seminoma. D40 expression is observed in several cancer cell lines and normal testis, which expression pattern reveals that D40 is one of the gene populations selectively expressed in cancer/testis (Immunol. Today 18, 267-8, 1997, The Cancer J. from Scientific American, 16-7, 1999).

**Table 5**

| Cancers | Positive incidents/total incidents tested |
|---|---|
| Oral cancer | 10/16(63%) |
| Uterine cancer | 8/18(44%) |
| Lung cancer | 8/20(40%) |
| Ovarian cancer | 4/11(36%) |
| Pancreatic cancer | 3/11(27%) |
| Neuroblastoma | 1/5(20%) |
| Colon tumor | 1/8(13%) |
| Cholangiocarcinoma | 0/7(0%) |
| Gastric cancer | 0/4(0%) |
| Seminoma | 0/3(0%) |

### B-5 (Immunity-inducing activity)

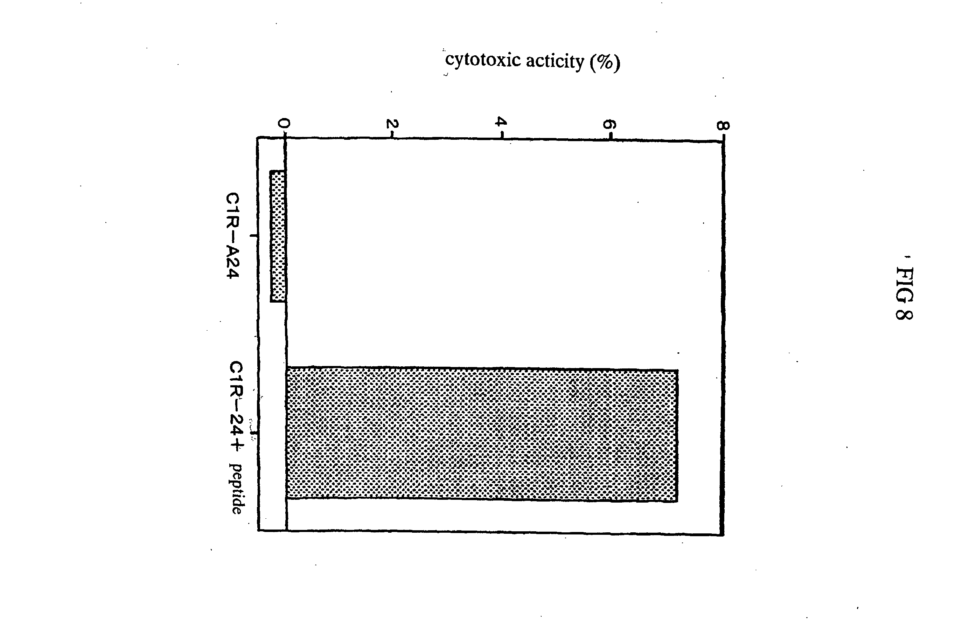

With the use of a peptide comprising the amino acid sequence shown by SEQ ID NO:36 (SYTIEINHRL) as a test peptide, the immunity-inducing activity of the peptide was examined by the cytotoxic test described in the Example A-10. Fig. 8 shows the result of cytotoxic activity of T cells derived from peripheral blood of a cancer patient. As is clear from Fig. 8, T cells derived from peripheral blood of a cancer patient (effecter cells) specifically injured target C1R-A24 cells which were added with 1 ìM of the peptide (SYTIEINHRL) and did not injure the control target C1R-A24 cells which were not added with the peptide (SYTIEINHRL). These indicate that the peptide (SYTIEINHRL) possesses the immunity-inducing activity.

### Industrial Applicability

Since HLA Class I molecules are not expressed in the testis, a D40-derived peptide and a HLA Class I molecule do not form a complex even when D40 is expressed so that D40 is not recognized by cytotoxic T lymphocytes. HLA Class I molecules are expressed whereas D40 is not in normal cells other than in the testis, so that it is again not recognized by cytotoxic T lymphocytes. However, since both D40 and HLA Class I molecules are expressed in cancer cells, the complex of these two are recognized by cytotoxic T lymphocytes and the cancer cells are injured. The present invention makes it possible to provide a cancer/testis antigen, with D40 as a primary example, which is applicable to diagnosis and immunotherapy for cancer, i.e. a cancer/testis antigen which is not expressed in normal tissues other than the testis, which is expressed in various cancers over a broad range and which induces immune response in a host, and the cancer/testis gene encoding the antigen or the like.

## Claims

1. A gene encoding the following protein (a) or (b).
(a) A protein comprising the amino acid sequence shown by SEQ ID NO:2.
(b) A protein which comprises an amino acid sequence wherein one or a few amino acids are deleted, substituted or added in the amino acid sequence shown by SEQ ID NO:2 and which has the immunity-inducing activity.

2. A gene encoding the following protein (a) or (b).
(a) A protein comprising the amino acid sequence shown by SEQ ID NO:3.
(b) A protein which comprises an amino acid sequence wherein one or a few amino acids are deleted, substituted or added in the amino acid sequence shown by SEQ ID NO:3 and which has the immunity-inducing activity.

3. DNA containing the base sequence shown by SEQ ID NO:1 or its complementary sequence and part of whole of these sequences.

4. DNA which hybridizes under a stringent condition with DNA of claim 3 that forms a gene, and which encodes a protein having the immunity-inducing activity.

5. A protein comprising the amino acid sequence shown by SEQ ID NO:2.

6. A protein which comprises an amino acid sequence wherein one or a few amino acids are deleted, substituted or added in the amino acid sequence shown by SEQ ID NO:2 and which has the immunity-inducing activity.

7. A protein comprising the amino acid sequence shown by SEQ ID NO:3.

8. A protein which comprises an amino acid sequence wherein one or a few amino acids are deleted, substituted or added in the amino acid sequence shown by SEQ ID NO:3 and which has the immunity-inducing activity.

9. A peptide which comprises part of the protein of any of claims 5 to 8 and which binds to a histocompatibility antigen molecule Class I.

10. The peptide according to claim 9, wherein said peptide comprises part of the protein of claim 5 or 6 and binds to a histocompatibility antigen molecule Class I.

11. The peptide according to claim 10, wherein said peptide comprises an amino acid sequence shown by any of SEQ ID NOs:4-111.

12. A peptide comprising the amino acid sequence of Ser-Tyr-Thr-Ile-Glu-Ile-Asn-His-Arg-Leu.

13. The peptide according to claim 9, wherein said peptide comprises part of the protein of claim 7 or 8 and binds to the histocompatibility antigen molecule Class I.

14. The peptide according to claim 13, wherein said peptide comprises an amino acid sequence shown by any of SEQ ID NOs:112-221.

15. A fusion protein or a fusion peptide wherein the protein of claim 5 or 6 or the peptide of any of claims 10 to 12, and a marker protein and/or a peptide tag, are bound.

16. A fusion protein or a fusion peptide wherein the protein of claim 7 or 8 or the peptide of claim 13 or 14, and a marker protein and/or a peptide tag, are bound.

17. An antibody which specifically binds to the protein of claim 5 or 6 or to the peptide of any of claims 10 to 12.

18. An antibody which specifically binds to the protein of claim 7 or 8 or to the peptide of claim 13 or 14.

19. The antibody according to claim 17 or 18, wherein said antibody is a monoclonal antibody.

20. A recombinant protein or peptide wherein the antibody of any of claims 17 to 19 specifically binds to said recombinant protein or peptide.

21. A host cell comprising an expression system capable of expressing the protein according to claim 5 or 6.

22. A host cell comprising an expression system capable of expressing the protein according to claim 7 or 8.

23. A non-human animal whose gene function to encode the protein of claim 5 or 6 is deficient on its chromosome.

24. A non-human animal whose gene function to encode the protein of claim 7 or 8 is deficient on its chromosome.

25. The non-human animal according to claim 23 or 24, wherein said non-human animal is a mouse or a rat.

26. A non-human animal which over-expresses the protein of claim 5 or 6.

27. A non-human animal which over-expresses the protein of claim 7 or 8.

28. The non-human animal according to claim 26 or 27, wherein said non-human animal is a mouse or a rat.

29. A method of screening a promoter or a suppressor for the immunity-inducing activity wherein the protein of claim 5 or 6, the peptide of any of claims 10 to 12 or a cell membrane expressing the protein of claim 5 or 6, and a test substance is used.

30. A method of screening a promoter or a suppressor for the immunity-inducing activity wherein the protein of claim 7 or 8, the peptide of claim 13 or 14, or a cell membrane expressing the protein of claim 7 or 8, and a test substance is used.

31. A method of screening a promoter or a suppressor for the immunity-inducing activity or for the expression of the protein of claim 5 or 6, wherein a cell expressing the protein and a test substance are used.

32. The method of screening a promoter or a suppressor for the immunity-inducing activity or for the expression of the protein of claim 5 or 6 according to claim 31, wherein the cell expressing the protein of claim 5 or 6 is the host cell of claim 21.

33. A method of screening a promoter or a suppressor for the immunity-inducing activity or for the expression of the protein of claim 7 or 8, wherein a cell expressing the protein and a test substance are used.

34. The method of screening a promoter or a suppressor for the immunity-inducing activity or for the expression of the protein of claim 7 or 8 according to claim 33, wherein the cell expressing the protein of claim 7 or 8 is the host cell of claim 22.

35. A method of screening a promoter or a suppressor for the immunity-inducing activity or a promoter or a suppressor for expression of the protein of any of claims 5 to 8, wherein the non-human animal of any of claims 23 to 25 and a test substance are used.

36. A method of screening a promoter or a suppressor for the immunity-inducing activity or a promoter or a suppressor for expression of the protein of any of claims 5 to 8, wherein the non-human animal of any of claims 26 to 28 and a test substance are used.

37. A promoter for the immunity-inducing activity obtained by the screening method according to any of claims 29 to 36.

38. A suppressor for the immunity-inducing activity obtained by the screening method according to any of claims 29 to 36.

39. An expression promoter for the protein of claim 5 or 6, wherein said expression promoter is obtained by the screening method according to any of claims 29 to 36.

40. An expression suppressor for the protein of claim 7 or 8, wherein said expression suppressor is obtained by the screening method according to any of claims 29 to 36.

41. An anti-tumor agent comprising the protein of any of claims 5 to 8, the peptide of any of claims 9 to 16, the protein or peptide of claim 20 or the antibody of any of claims 17 to 19, as an active ingredient.

42. The anti-tumor agent according to claim 41, wherein the cancer is one or more cancers selected from uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma and colon cancer.

43. A method for detecting a cytotoxic T cell or its precursor cell wherein a HLA molecule, and the peptide of any of claims 9 to 16 or the peptide of claim 20 are used.

44. A method for detecting a cytotoxic T cell or its precursor cell wherein a complex of a HLA Class I molecule, and the peptide of any of claims 9 to 16 or the peptide of claim 20 is formed on the surface of fluorescent microparticles.

45. A detection reagent for a cytotoxic T cell or its precursor cell, wherein said detection reagent comprises a HLA molecule, and the peptide of any of claims 9 to 16 or the peptide of claim 20.

46. A detection reagent for a cytotoxic T cell or its precursor cell wherein said detection reagent comprises: a complex of a HLA Class I molecule, and the peptide of any of claims 9 to 16 or the peptide of claim 20; and a fluorescent microparticle.

47. A cytotoxic T cell induced by in vitro stimuli wherein the protein of any of claims 5 to 8, the peptide of any of claims 9 to 16, or the protein or peptide of claim 20 is used.

48. A diagnostic probe for cancer comprising whole or part of the antisense strand of DNA or RNA encoding the protein of claim 5 or 6.

49. A diagnostic probe for cancer comprising whole or part of the antisense strand of DNA or RNA encoding the protein of claim 7 or 8.

50. A diagnostic drug for cancer comprising the diagnostic probe for cancer according to claim 48 or 49 and/or the antibody according to any of claims 17 to 19.

51. The diagnostic drug for cancer according to claim 50, wherein the cancer is one or more cancers selected from uterine cervix cancer, epithelial-like cancer, T cell tumor, promyelocytic leukemia, esophageal cancer, pancreatic cancer, malignant melanoma, lung cancer, oral cancer, breast cancer, bladder cancer, uterine cancer, ovarian cancer, neuroblastoma and colon cancer.
